(19) **Europäisches Patentamt / European Patent Office / Office européen des brevets**

(11) **EP 3 580 357 B1**

(12) **EUROPEAN PATENT SPECIFICATION**

(45) Date of publication and mention of the grant of the patent:
**27.04.2022 Bulletin 2022/17**

(21) Application number: **18707504.9**

(22) Date of filing: **12.02.2018**

(51) International Patent Classification (IPC):
**C12Q 1/6809** (2018.01)   **C12Q 1/6886** (2018.01)

(52) Cooperative Patent Classification (CPC):
(C-Sets available)
**C12Q 1/6886; C12Q 1/6809;** C12Q 2600/118;
C12Q 2600/158   (Cont.)

(86) International application number:
**PCT/US2018/017790**

(87) International publication number:
**WO 2018/148642 (16.08.2018 Gazette 2018/33)**

(54) **ALGORITHMS AND METHODS FOR ASSESSING LATE CLINICAL ENDPOINTS IN PROSTATE CANCER**

ALGORITHMEN UND VERFAHREN ZUR BEURTEILUNG VON SPÄTEN KLINISCHEN ENDPUNKTEN BEI PROSTATAKREBS

ALGORITHMES ET PROCÉDÉS POUR ÉVALUER DES CRITÈRES CLINIQUES TARDIFS DANS LE CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2017   US 201762458474 P**
**17.03.2017   US 201762473204 P**
**30.10.2017   US 201762578622 P**

(43) Date of publication of application:
**18.12.2019   Bulletin 2019/51**

(73) Proprietor: **Genomic Health, Inc.**
**Redwood City, CA 94063 (US)**

(72) Inventors:
• **LU, Ruixiao**
  **Fremont,**
  **CA 94539 (US)**
• **CRAGER, Michael**
  **Redwood City**
  **California 94063 (US)**
• **ZHANG, Nan**
  **Palo Alto,**
  **California 94306 (US)**
• **MADDALA, Tara**
  **Sunnyvale**
  **California 94087 (US)**
• **FEBBO, Phillip**
  **Mill Valley**
  **California 94941 (US)**
• **LAWRENCE, Hugh Jeffrey**
  **Oakland**
  **California 94618 (US)**

(74) Representative: **Forresters IP LLP**
**Skygarden**
**Erika-Mann-Straße 11**
**80636 München (DE)**

(56) References cited:
**WO-A1-2016/193109     US-A1- 2013 196 321**

• **ERIC A. KLEIN ET AL: "A 17-gene Assay to Predict Prostate Cancer Aggressiveness in the Context of Gleason Grade Heterogeneity, Tumor Multifocality, and Biopsy Undersampling", EUROPEAN UROLOGY, vol. 66, no. 3, 1 September 2014 (2014-09-01), pages 550-560, XP055466464, AMSTERDAM, NL ISSN: 0302-2838, DOI: 10.1016/j.eururo.2014.05.004**

• JENNIFER CULLEN ET AL: "A Biopsy-based 17-gene Genomic Prostate Score Predicts Recurrence After Radical Prostatectomy and Adverse Surgical Pathology in a Racially Diverse Population of Men with Clinically Low- and Intermediate-risk Prostate Cancer", EUROPEAN UROLOGY, vol. 68, no. 1, 1 July 2015 (2015-07-01), pages 123-131, XP055466468, AMSTERDAM, NL ISSN: 0302-2838, DOI: 10.1016/j.eururo.2014.11.030

(52) Cooperative Patent Classification (CPC): (Cont.)

C-Sets
**C12Q 1/6809**

**Description**

## TECHNICAL FIELD

The present disclosure relates to uses of a multiple gene-expression based Genomic Prostate Score™ (GPS™) test algorithm for assessment of various clinical endpoints in prostate cancer patients, such as risks of clinical recurrence (CR) also referred to herein as metastasis, biochemical recurrence (BCR), distant metastasis (Mets), and prostate cancer death (PCD) and, in some embodiments, for determining clinical management options for low and intermediate risk prostate cancer patients.

## INTRODUCTION

The introduction of prostate-specific antigen (PSA) screening in 1987 has led to the diagnosis and aggressive treatment of many cases of indolent prostate cancer that would never have become clinically significant or caused death. The reason for this is that the natural history of prostate cancer in the majority of cases are indolent and even if untreated, would not progress during the course of a man's life to cause suffering or death. While approximately half of men develop invasive prostate cancer during their lifetimes (as detected by autopsy studies) (B. Halpert et al, Cancer 16: 737-742 (1963); B. Holund, Scand J Urol Nephrol 14: 29-35 (1980); S. Lundberg et al., Scand J Urol Nephrol 4: 93-97 (1970); M. Yin et al., J Urol 179: 892-895 (2008)), only 17% will be diagnosed with prostate cancer and only 3% will die as a result of prostate cancer. Cancer Facts and Figures. Atlanta, GA: American Cancer Society (2010); JE Damber et al., Lancet 371: 1710-1721 (2008).

However, currently, a high percentage of men who are diagnosed with prostate cancer, even low-risk prostate cancer, are treated with either immediate radical prostatectomy (RP) or definitive radiation therapy. MR Cooperberg et al., J Clin Oncol 28: 1117-1123 (2010); MR Cooperberg et al., J Clin Oncol 23: 8146-8151 (2005). Surgery and radiation therapy reduce the risk of recurrence and death from prostate cancer (AV D'Amico et al., Jama 280: 969-974 (1998); M Han et al., Urol Clin North Am 28: 555-565 (2001); WU Shipley et al., Jama 281: 1598-1604 (1999); AJ Stephenson et al., J Clin Oncol 27: 4300-4305 (2009)), however estimates of the number of men that must be treated to prevent one death from prostate cancer range from 12 to 100. A Bill-Axelson et al., J Natl Cancer Inst 100: 1144-1154 (2008); J Hugosson et al., Lancet Oncol 11: 725-732 (2010); LH Klotz et al., Can J Urol 13 Suppl 1: 48-55 (2006); S Loeb et al., J Clin Oncol 29: 464-467 (2011); FH Schroder et al., N Engl J Med 360: 1320-1328 (2009). This over-treatment of prostate cancer comes at a cost of money and toxicity. For example, the majority of men who undergo radical prostatectomy suffer incontinence and impotence as a result of the procedure (MS Litwin et al., Cancer 109: 2239-2247 (2007); MG Sanda et al., N Engl J Med 358: 1250-1261 (2008), and as many as 25% of men regret their choice of treatment for prostate cancer. FR Schroeck et al., Eur Urol 54: 785-793 (2008).

One of the reasons for the over-treatment of prostate cancer is the lack of adequate prognostic tools to distinguish men who need immediate definitive therapy from those who are appropriate candidates to defer immediate therapy and undergo active surveillance instead. For example, of men who appear to have low-risk disease based on the results of clinical staging, pre-treatment PSA, and biopsy Gleason score, and have been managed with active surveillance on protocols, 30-40 % experience disease progression (diagnosed by rising PSA, an increased Gleason score on repeat biopsy, or clinical progression) over the first few years of follow-up, and some of them may have lost the opportunity for curative therapy. HB Carter et al., J Urol 178: 2359-2364 and discussion 2364-2355 (2007); MA Dall'Era et al., Cancer 112: 2664-2670 (2008); L Klotz et al., J Clin Oncol 28: 126-131 (2010). Also, of men who appear to be candidates for active surveillance, but who undergo immediate prostatectomy anyway, 30-40% are found at surgery to have higher risk disease than expected as defined by having high-grade (Gleason score of 3+4 or higher) or non-organ-confined disease (extracapsular extension (ECE) or seminal vesicle involvement (SVI)). SL et al., J Urol 181: 1628-1633 and discussion 1633-1624 (2009); CR Griffin et al., J Urol 178: 860-863 (2007); PW Mufarrij et al., J Urol 181: 607-608 (2009).

Estimates of recurrence risk and treatment decisions in prostate cancer are currently based primarily on PSA levels and/or clinical tumor grading and stage. Although clinical tumor stage has been demonstrated to have a significant association with outcome, sufficient to be included in pathology reports, the College of American Pathologists Consensus Statement noted that variations in approach to the acquisition, interpretation, reporting, and analysis of this information exist. C. Compton, et al., Arch Pathol Lab Med 124:979-992 (2000). As a consequence, existing pathologic staging methods have been criticized as lacking reproducibility and therefore may provide imprecise estimates of individual patient risk.

To provide further information to help determine likelihood of clinical outcome, studies have been conducted to look for gene expression markers that may predict likelihood of clinical recurrence, and algorithms have been developed and commercialized that assess, for example, expression levels of multiple genes. E. Klein et al., Eur Urol 66: 550-560 (2014); J. Cullen, et al., Eur Urol 68: 123-131 (2015); International Patent Publication No. WO 2013/116144. The present disclosure relates to methods of using an assay measuring expression levels of at least 12 different genes from several gene subsets, for example, as a means of determining, for patients placed into a very low, low, intermediate, or high risk group on the basis of other parameters, their relative risks of certain longer term events such as clinical recurrence (CR), biochemical recurrence (BCR), distant metastases (Mets), and protstate cancer death (PCD). In some embodiments, a patient's Genomic Prostate Score (GPS) result, combined with his clinical and pathologic features, places him in a different risk category to his original clinical risk group. In some embodiments, this further refines and individualizes a patient's estimated risk for aggressive disease and allows for improved treatment plans for patients. US 2013/196321 A1 discloses a method for predicting a likelihood of a clinical outcome for a patient with prostate cancer.

## SUMMARY

The invention is set out in the appended set of claims. The present disclosure, in some embodiments, includes methods of predicting likelihood of adverse clinical outcome in a prostate cancer patient, such as BCR, Mets, and PCD, comprising: (a) measuring, in a biological sample containing cancer cells obtained from the patient, levels of RNA transcripts of the following genes: BGN, COL1A1, SFRP4, FLNC, GSN, TPM2, GSTM2, FAM13C, KLK2, AZGP1, SRD5A2, and TPX2; (b) normalizing the levels of the RNA transcripts of the genes to obtain normalized gene expression levels; (c) calculating a quantitative score (QS) for the patient, such as a GPS result as described herein; (d) assigning the patient to a quantitative score group, wherein (i) the patient is assigned to a lower score group if the patient's QS is either < or ≤ a threshold of 38, 39, 40, 41, or 42; and (ii) the patient is assigned to a high score group if the patient's QS is either > or ≥ a threshold of 38, 39, 40, 41, or 42; and optionally (e) predicting risk of an adverse clinical outcome for the patient such as CR, BCR, Mets, and PCD, based upon the patient's score group, wherein a lower score group indicates a lower risk of adverse clinical outcome than a high score group. In some embodiments, in part (d), the patient is assigned to a lower score group if the patient's QS is either < or ≤ 40; and (ii) the patient is assigned to a high score group if the patient's QS is either > or ≥ 40. In some embodiments, in part (d), the patient is assigned to a lower score group if the patient's QS is ≤ 40; and (ii) the patient is assigned to a high score group if the patient's QS is > 40. In some embodiments, for a patient in the lower score group of part (d)(i), the patient is assigned to a low score group if the patient's QS is either < or ≤ a further threshold of 18, 19, 20, 21, or 22 and is assigned to an intermediate score group if the patient's QS is either > or ≥ a threshold of 18, 19, 20, 21, or 22 and if the patient does not fall within the high score group. In some embodiments, for a patient in the lower score group of part (d)(i), the patient is assigned to a low score group if the patient's QS is either < or ≤ 20 and is assigned to an intermediate score group if the patient's QS is either > or ≥ 20 and if the patient does not fall within the high score group. In some embodiments, for a patient in the lower score group of part (d)(i), the patient is assigned to a low score group if the patient's QS is ≤ 20 and is assigned to an intermediate score group if the patient's QS is > 20 and if the patient does not fall within the high score group. Thus, in some embodiments, the patients are placed into the following three groups: QS< 20, QS < 20 but <40, and QS > 40. In any of the embodiments, the QS may be a GPS as described herein.

In some embodiments of the above methods, the patient is a very low or low, intermediate, or high risk patient. In some such embodiments, the patient is a very low or low, intermediate, or high risk patient according to one or both of the AUA/EAU or NCCN classifications. In some embodiments, the method further comprises providing a report providing the patient's quantitative score and score group. In some embodiments, the levels of the RNA transcripts are normalized against at least one reference gene chosen from GUS, ARF1, ATP5E, CLTC, GPS1, and PGK1. In some embodiments, the biological sample is a fresh, frozen, or a fixed, paraffin-embedded sample. In some embodiments, the levels of the RNA transcripts are determined using quantitative reverse-transcriptase polymerase chain reaction (RT-PCR). In some embodiments, the method further comprises determining treatment for the patient based on the patient's quantitative score group. In some embodiments, the adverse clinical outcome is one or more of clinical recurrence (CR), biochemical recurrence (BCR), distant metastasis (Mets), or prostate cancer death (PCD).

The present disclosure also encompasses methods of assigning a relative risk of adverse clinical outcome to a low or intermediate risk prostate cancer patient, comprising: (a) measuring, in a biological sample containing cancer cells obtained from the patient, levels of RNA transcripts of the following genes: BGN, COL1A1, SFRP4, FLNC, GSN, TPM2, GSTM2, FAM13C, KLK2, AZGP1, SRD5A2, and TPX2; (b) normalizing the levels of the RNA transcripts of the genes to obtain normalized gene expression levels; (c) calculating a quantitative score (QS) for the patient, such as a GPS result as described herein; and (d) assigning the patient to a quantitative score group, wherein (i) the patient is assigned to a lower score group if the patient's QS is either < or ≤ a threshold of 38, 39, 40, 41, or 42; and (ii) the patient is assigned to a high score group if the patient's QS is either > or ≥ a threshold of 38, 39, 40, 41, or 42. In some embodiments, in part (d), the patient is assigned to a lower score group if the patient's QS is either < or ≤ 40; and (ii) the patient is assigned to a high score group if the patient's QS is either > or ≥ 40. In some embodiments, in part (d), the patient is assigned to a lower score group if the patient's QS is ≤ 40; and (ii) the patient is assigned to a high score group if the patient's QS is > 40. In some embodiments, for a patient in the lower score group of part (d)(i), the patient is assigned to a low score group if the patient's QS is either < or ≤ a further threshold of 18, 19, 20, 21, or 22 and is assigned to an intermediate score group if the patient's QS is either > or ≥ a threshold of 18, 19, 20, 21, or 22 and if the patient does not fall within the high score group. In some embodiments, for a patient in the lower score group of part (d)(i), the patient is assigned to a low score group if the patient's QS is either < or ≤ 20 and is assigned to an intermediate score group if the patient's QS is either > or ≥ 20 and if the patient does not fall within the high score group. In some embodiments, for a patient in the lower score group of part (d)(i), the patient is assigned to a low score group if the patient's QS is ≤ 20 and is assigned to an intermediate score group if the patient's QS is > 20 and if the patient does not fall within the high score group. In some embodiments, the QS is a GPS as described herein.

In some embodiments, the patient is an intermediate risk patient. In some such embodiments, the patient is an intermediate risk patient according to one or both of the AUA/EAU or NCCN classifications. In some embodiments, the method further comprises providing a report providing the patient's quantitative score and score group. In some embodiments, the levels of the RNA transcripts are normalized against at least one reference gene chosen from GUS, ARF1, ATP5E, CLTC, GPS1, and PGK1. In some embodiments, the biological sample is a fresh, frozen, or a fixed, paraffin-embedded sample. In some embodiments, the levels of the RNA transcripts are determined using quantitative reverse-transcriptase polymerase chain reaction (RT-PCR). In some embodiments, the method further comprises determining treatment for the patient based on the patient's quantitative score group. In some embodiments, the patient is an intermediate risk patient and, if the patient is in the high score group, the method further comprises refining the risk estimate for the patient as similar to a high risk patient, and optionally, if the patient is in the lower score group, the method comprises maintaining the patient's classification as an intermediate risk patient. In some embodiments the method further comprises, if the patient is in the high score group, treating the patient with multi-modal therapy or with a standard therapy for a high risk patient. In some such embodiments, multi-modal therapy comprises (a) administration of at least one hormonal therapy agent and/or (b) administration of at least one immunotherapy agent, and/or (c) administration of at least one chemotherapy agent and/or (d) surgery, and/or (e) radiation, that is, any combination of (a)-(e). In some embodiments, if the patient is in the low score group, the method further comprises treating or managing the patient with active surveillance.

The present disclosure also encompasses methods of treating an intermediate risk prostate cancer patient determined to have a quantitative score according to the methods above in the high score group, comprising administering multi-modal therapy to the patient. In some embodiments, the multi-modal therapy comprises (a) administration of at least one hormonal therapy agent and/or (b) administration of at least one immunotherapy agent, and/or (c) administration of at least one chemotherapy agent and/or (d) surgery, and/or (e) radiation, and/or (f) any combination of (a)-(e).

## DESCRIPTIONS OF THE DRAWINGS

**Fig. 1A** provides a graph showing the proportion of prostate cancer subjects who remained metastasis free over a 20 year period within each of four NCCN (National Clinical Practice Guidelines in Oncology) risk groups (very low, low, intermediate, and high). There were 259 total subjects analyzed. **Fig. 1B** shows the proportion of the subjects in those four groups who did not experience prostate cancer death (PCD) over the same 20-year period.

**Fig. 2A** shows the distribution of the 259 prostate cancer subjects in the NCCN risk groups and provides the number of subjects in each of the very low + low risk, intermediate risk, and high risk groups, and shows the range and the median Global Prostate Score (GPS) for each of the groups. **Fig. 2B** shows the distribution of androgen group scores in each of the NCCN risk groups.

**Fig. 3** shows the 10-year risk of metastasis against GPS in the very low + low, intermediate, and high NCCN risk grops.

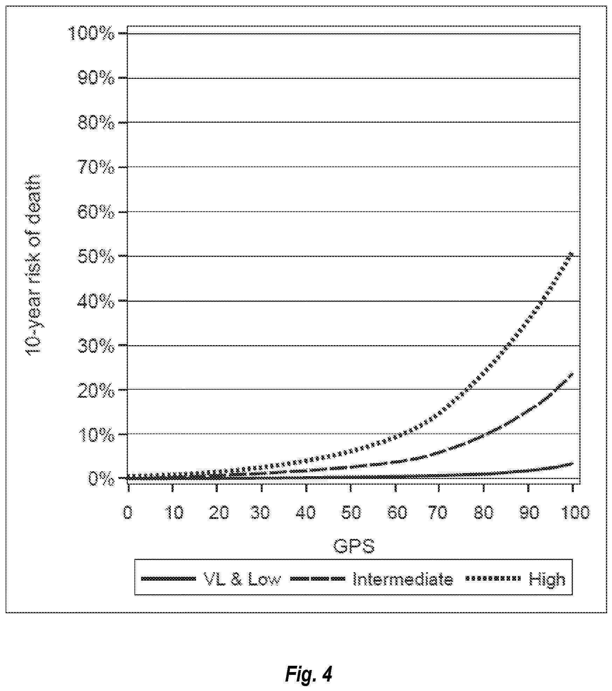

**Fig. 4** shows the 10-year risk of death against GPS in the very low + low, intermediate, and high NCCN risk grops.

**Fig. 5** shows the standardized hazard ratios for the GPS and the underlying stromal, cellular organization, androgen, and proliferation group scores associated with prediction of metastasis (Mets; left panel) and prostate cancer death (PCD; right panel).

## DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described herein. For purposes of the invention, the following terms are defined below.

The terms "tumor" and "lesion" as used herein, refer to all neoplastic cell growth and proliferation, whether malignant or benign, and all pre-cancerous and cancerous cells and tissues. Those skilled in the art will realize that a tumor tissue sample may comprise multiple biological elements, such as one or more cancer cells, partial or fragmented cells, tumors in various stages, surrounding histologically normal-appearing tissue, and/or macro or micro-dissected tissue.

The terms "cancer" and "cancerous" refer to or describe the physiological condition in mammals that is typically characterized by unregulated cell growth. Examples of cancer in the present disclosure include cancer of the urogenital tract, such as prostate cancer.

As used herein, the term "prostate cancer" is used in the broadest sense and refers to all stages and all forms of cancer arising from the tissue of the prostate gland.

Staging of the cancer assists a physician in assessing how far the disease has progressed and to plan a treatment for the patient. Staging may be done clinically (clinical staging) by physical examination, blood tests, or response to radiation therapy, and/or pathologically (pathologic staging) based on surgery, such as radical prostatectomy. According to the tumor, node, metastasis (TNM) staging system of the American Joint Committee on Cancer (AJCC), AJCC Cancer Staging Manual (7th Ed., 2010), the various stages of prostate cancer are defined as follows: Tumor: TI: clinically inapparent tumor not palpable or visible by imaging, T1a: tumor incidental histological finding in 5% or less of tissue resected, T1b: tumor incidental histological finding in more than 5% of tissue resected, T1c: tumor identified by needle biopsy; T2: tumor confined within prostate, T2a: tumor involves one half of one lobe or less, T2b: tumor involves more than half of one lobe, but not both lobes, T2c: tumor involves both lobes; T3: tumor extends through the prostatic capsule, T3a: extracapsular extension (unilateral or bilateral), T3b: tumor invades seminal vesicle(s); T4: tumor is fixed or invades adjacent structures other than seminal vesicles (bladder neck, external sphincter, rectum, levator muscles, or pelvic wall). Generally, a clinical T (cT) stage is T1 or T2 and pathologic T (pT) stage is T2 or higher. Node: NO: no regional lymph node metastasis; NI: metastasis in regional lymph nodes. Metastasis: M0: no distant metastasis; MI: distant metastasis present.

The Gleason Grading system is used to help evaluate the prognosis of men with prostate cancer. Together with other parameters, it is incorporated into a strategy of prostate cancer staging, which predicts prognosis and helps guide therapy. A Gleason "score" or "grade" is given to prostate cancer based upon its microscopic appearance. Tumors with a low Gleason score typically grow slowly enough that they may not pose a significant threat to the patients in their lifetimes. These patients may be monitored by "watchful waiting" or "active surveillance" over time. Cancers with a higher Gleason score may be more aggressive and have a worse prognosis, and these patients are generally treated with surgery (e.g., radical prostatectomy) and, in some cases, other therapy (e.g., radiation, hormone, ultrasound, chemotherapy). Gleason scores (or sums) comprise grades of the two most common tumor patterns. These patterns are referred to as Gleason patterns 1-5, with pattern 1 being the most well-differentiated. Most have a mixture of patterns. To obtain a Gleason score or grade, the dominant pattern is added to the second most prevalent pattern to obtain a number between 2 and 10. The Gleason Grades are as followsas: GGG1 (GS≤6), GGG2 (GS 3+4=7), GGG3 (GS 4+3=7), GGG4 (GS 4+4=8, GS 3+5=8, GS 5+5=8) and GGG5 (GS 9 or 10).

Stage groupings: Stage I: TIa N0 M0 G1; Stage II: (T1a N0 M0 G2-4) or (T1b, c, T1, T2, NO M0 Any G); Stage III: T3 NO M0 Any G; Stage IV: (T4 NO M0 Any G) or (Any T N1 M0 Any G) or (Any T Any N M1 Any G).

The term "upgrading" as used herein refers to an increase in Gleason grade determined from biopsy to Gleason grade determined from radical prostatectomy (RP). For example, upgrading includes a change in Gleason grade from 3+3 or 3+4 on biopsy to 3+4 or greater on RP. "Significant upgrading" or "upgrade 2" as used herein, refers to a change in Gleason grade from 3+3 or 3+4 determined from biopsy to 4+3 or greater, or seminal vessical involvement (SVI), or extracapsular involvement (ECE) as determined from RP.

The term "high grade" as used herein refers to Gleason score of >=3+4 or >=4+3 on RP. The term "low grade" as used herein refers to a Gleason score of 3+3 on RP. In a particular embodiment, "high grade" disease refers to Gleason score of at least major pattern 4, minor pattern 5, or tertiary pattern 5.

The term "upstaging" as used herein refers to an increase in tumor stage from biopsy to tumor stage at RP. For example, upstaging is a change in tumor stage from clinical T1 or T2 stage at biopsy to pathologic T3 stage at RP.

The term "non organ-confined disease" as used herein refers to having pathologic stage T3 disease at RP. The term "organ-confined" as used herein refers to pathologic stage pT2 at RP. The term "high-grade or non-organ-confined disease" refers to prostate cancer with a Gleason score of at least major pattern 4, minor pattern 5, or tertiary pattern 5, or pathologic stage T3.

The term "adverse pathology" or "AP" as used herein refers to a high grade disease as defined above, or non organ-confined disease as defined above. In a particular embodiment, "adverse pathology" refers to prostate cancer with a Gleason score of >=3+4 or >=4+3 or GS>4+3 and/or pathologic stage T3.

Prostate cancer patients may be placed into particular "risk groups" or "risk classifications" based upon certain recognized risk classification systems provided by the American Urological Association (AUA) or the National Clinical Practice Guidelines in Oncology (NCCN) or to the UCSF-developed Cancer of the Prostate Risk Assessment (CAPRA) score system. Thus, in general, the term "risk classification" or "risk group" means a grouping of subjects based on a set of prognostic factors such as PSA level, Gleason score, and clinical stage, and the like, that have been classified to have a similar level of risk of negative clinical outcomes, such as low, medium, or high.

For example, under the AUA 2007 guidelines, a "low risk" patient is one who has a prostate antigen (PSA) level of 10 ng/mL or less, a Gleason score of 6 or less and clinical stage of T1c or T2a. An AUA "high risk" patient has a PSA of > 20 ng/mL, or a Gleason score of 8-10, or a clinical stage of T2c. An AUA "ntermediate risk" patient has a PSA of from > 10 ng/mL to 20 ng/mL, or a Gleason score of 7, or a clinical stage of T2b, but who does not satisfy any of the "high risk" conditions. Under the NCCN guidelines for prostate cancer (Version 2.2017), a "very low risk" patient has a stage of T1c, a Gleason score of less than or equal to 6, a PSA of less than 10 ng/mL, a PSA density of less than 0.15 ng/mL/g, and fewer than 3 prostate biopsy cores that are positive, with less than or equal to 50% cancer in each core. An NCCN "low risk patient has a clinical stage of T1-T2a, a gleason score of less than or equal to 6, and a PSA of less than 10 ng/mL. An NCCN "high risk" patient has a clinical stage of T3a, or a Gleason score of 8-10, or PSA of > 20 ng/mL. An NCCN "intermediate risk" patient has a clinical grade of T2b-T2c, or a Gleason score of 7, or PSA from 10-20 ng/mL. The CAPRA score is calculated from age at diagnosis, PSA at diagnosis, Gleason score of the biopsy, clinical stage, and percent of the biopsy cores involved with cancer and a point score is assigned to each variable to obtain a resulting score. A CAPRA score of 0-2 indicates low risk, a score of 3-5 indicates intermediate risk, and a score of 6-10 indicates high risk. Reference to, for example, an "intermediate risk" patient herein without giving the scoring system used (e.g. AUA, NCCN, or CAPRA) means a patient falling within the intermediate risk group of at least one of those systems. Similarly, reference to a "low risk" patient without reference to a particular system means a patient falling within the low or very low risk group of at least one of those systems. Reference to a "high risk" patient without reference to a particular system means a patient falling within the high risk group of at least one of those systems.

A "standard therapy" as used herein, such as a standard therapy for a high risk patient or a low risk patient, refers to one or more types of therapy recommended by bodies such as AUA or NCCN for such patients.

As used herein, the terms "active surveillance" and "watchful waiting" both comprise closely monitoring a patient's condition without giving any treatment until symptoms appear or change. The term "watchful waiting" encompasses a forgoing of definitive treatment of the primary prostate tumor and provision of only palliative treatment for local or metastatic progression if that occurs, such as transurethral resection of the prostate, management of urinary tract obstruction, hormonal therapy, and radiotherapy for palliation of metastatic lesions. The term "active surveillance" means a regular clinical monitoring program for the patient that does not include initial surgical, radiation or drug treatment, with a goal of monitoring the patient for any subsequent changes that suggest a need for definitive treatment such as surgical, radiation and/or drug treatment. Active surveillance encompasses, for example, periodic PSA testing, periodic biopsies, and other periodic tests designed to assess tumor stage and risk of tumor progression.

As used herein, the term "surgery" applies to surgical methods undertaken for removal of cancerous tissue, including pelvic lymphadenectomy, radical prostatectomy (RP), transurethral resection of the prostate (TURP), excision, dissection, and tumor biopsy/removal. The tumor tissue or sections used for gene expression analysis may have been obtained from any of these methods.

As used herein, the terms "biological sample containing cancer cells" or "biological sample containing tumor cells" refer interchangeably to a sample comprising tumor material obtained from a cancer patient. The term encompasses tumor tissue samples, for example, tissue obtained by radical prostatectomy and tissue obtained by biopsy, such as for example, a core biopsy or a fine needle biopsy. The biological sample may be fresh, frozen, or a fixed, wax-embedded tissue sample, such as a formalin-fixed, paraffin-embedded tissue sample. A biological sample also encompasses bodily fluids containing cancer cells, such as blood, plasma, serum, urine, and the like. Additionally, the term "biological sample containing cancer cells" encompasses a sample comprising tumor cells obtained from sites other than the primary tumor, e.g., circulating tumor cells. The term also encompasses cells that are the progeny of the patient's tumor cells, e.g. cell culture samples derived from primary tumor cells or circulating tumor cells. The term further encompasses samples that may comprise protein or nucleic acid material shed from tumor cells *in vivo*, e.g., bone marrow, blood, plasma, serum, and the like. The term also encompasses samples that have been enriched for tumor cells or otherwise manipulated after their procurement and samples comprising polynucleotides and/or polypeptides that are obtained from a patient's tumor material.

The term "prognosis" is used herein to refer to the likelihood that a cancer patient will have a cancer-attributable death or progression, including recurrence, metastatic spread, and drug resistance, of a neoplastic disease such as prostate cancer. For example, a "good prognosis" would include long term survival without recurrence and a "bad prognosis" would include cancer recurrence.

The term "recurrence" is used herein to refer to local or distant recurrence (i.e., distant metastasis) of cancer and encompasses both "clinical recurrence" and "biochemical recurrence."

The term "clinical recurrence" or "CR" refers to a recurrence such as either local recurrence or distant metastasis as detected, for example, in a follow-up biopsy or other clinical procedure.

The term "biochemical recurrence" or "BCR" refers to recurrence as detected on the basis of a change in a biochemical marker such as PSA. In some embodiments, an initial post-surgical PSA level of $\geq$ 0.2 ng/mL followed by a confirmatory PSA level of > 0.2 ng/mL in a subsequent test indicates BCR.

The term "prostate cancer death" or "PCD" refers to death of a patient attributed to prostate cancer, including recurrence of an earlier-identified prostate cancer in the patient.

The term "distant metastasis" or "Mets" refers to recurrence of cancer at a site distant from the original prostate tumor, such as in bone or in one or more distant lymph nodes or in another non-prostate organ or tissue.

The term "clinical recurrence-free interval (cRFI)" is used herein as time from surgery to first clinical recurrence or death due to clinical recurrence of prostate cancer. If follow-up ended without occurrence of clinical recurrence, or other primary cancers or death occurred prior to clinical recurrence, time to cRFI is considered censored; when this occurs, the only information known is that up through the censoring time, clinical recurrence has not occurred in this subject. Biochemical recurrences are ignored for the purposes of calculating cRFI.

The term "biochemical recurrence-free interval (bRFI)" is used herein to mean the time from surgery to first biochemical recurrence of prostate cancer. If clinical recurrence occurred before biochemical recurrence, follow-up ended without occurrence of bRFI, or other primary cancers or death occurred prior to biochemical recurrence, time to biochemical recurrence is considered censored at the first of these.

In practice, the calculation of the time-to-event measures listed above may vary from study to study depending on the definition of events to be considered censored.

As used herein, the term "expression level" or "level" of a gene herein refers to the level of expression of an RNA transcript of the gene or of its polypeptide translation product. As used herein, the term "normalized level" or "normalized expression level" of a gene herein refers to the level of expression of an RNA transcript of the gene or of its polypeptide translation product after normalization against the expression level of one or more reference genes herein.

The term "gene product" or "expression product" are used herein to refer to the RNA (ribonucleic acid) transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA.

Unless indicated otherwise, each gene name used herein corresponds to the Official Symbol assigned to the gene and provided by Entrez Gene (URL: www (dot) ncbi (dot) nlm (dot) gov (slash) sites (slash) entrez) as of the filing date of this application.

The term "microarray" refers to an ordered arrangement of hybridizable array elements, e.g. oligonucleotide or polynucleotide probes, on a substrate.

The term "polynucleotide" generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons, are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNArDNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including in vitro recombinant DNA-mediated techniques and by expression of DNAs in cells and organisms.

The term "Ct" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The term "Cp" as used herein refers to "crossing point." The Cp value is calculated by determining the second derivatives of entire qPCR amplification curves and their maximum value. The Cp value represents the cycle at which the increase of fluorescence is highest and where the logarithmic phase of a PCR begins.

The term "thresholding" refers to a procedure used to account for non-linear relationships between gene expression measurements and clinical response as well as to further reduce variation in reported patient scores. When thresholding is applied, all measurements below or above a threshold value are set to that threshold value. A non-linear relationship between gene expression and outcome could be examined using smoothers or cubic splines to model gene expression on recurrence free interval using Cox PH regression or on adverse pathology status using logistic regression. D. Cox, Journal of the Royal Statistical Society, Series B 34:187-220 (1972). Variation in reported patient scores could be examined as a function of variability in gene expression at the limit of quantitation and/or detection for a particular gene.

As used herein, the term "amplicon," refers to pieces of DNA that have been synthesized using amplification techniques, such as polymerase chain reactions (PCR) and ligase chain reactions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology (Wiley Interscience Publishers, 1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-500C. The skilled artisan will recognize how to adjust the temperature, ionic strength, etc. as necessary to accommodate factors such as probe length and the like.

The terms "splicing" and "RNA splicing" are used interchangeably and refer to RNA processing that removes introns and joins exons to produce mature mRNA with continuous coding sequence that moves into the cytoplasm of an eukaryotic cell.

The terms "correlated" and "associated" are used interchangeably herein to refer to the association, either between two measured or calculated entities or alternatively between a measured or calculated entity (e.g. Gleason score, PSA level, GPS result) and an event (e.g. CR, PCD).

A "cartridge" refers to a physical structure that contains reagents for processing a sample, such as reagents for detecting RNA transcript levels from a sample. In some embodiments, reactions such as thermocycling and sample treatment such as RNA extraction can take place within a cartridge. A "well" comprised within a cartridge may be a chamber, indentation, specific surface, or other type of specific area that may hold particular reagents such as primers and/or probes for performing PCR or other chemical reactions.

A "computer-based system" refers to a system of hardware, software, and data storage medium used to analyze information. The minimum hardware of a patient computer-based system comprises a central processing unit (CPU), and hardware for data input, data output (e.g., display), and data storage. An ordinarily skilled artisan can readily appreciate that any currently available computer-based systems and/or components thereof are suitable for use in connection with the methods of the present disclosure. The data storage medium may comprise any manufacture comprising a recording of the present information as described above, or a memory access device that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

## ALGORITHM-BASED METHODS, THE GPS ALGORITHM, AND GENE SUBSETS

The present invention provides an algorithm-based molecular diagnostic assay for determining the relative risks of particular clinical outcomes for a patient with prostate cancer and for assigning patients to particular treatment groups based on the score obtained from the assay.

In some embodiments, this disclosure relates to methods of obtaining a quantitative score based on the expression level of each of the following genes: BGN, COL1A1, SFRP4, FLNC, GSN, GSTM2, TPM2, TPX2, AZGP1, FAM13C, KLK2, and SRD5A2, and one or more reference genes. In some embodiments, the quantitative score is scaled to a range of 0 to 100 to obtain a Global Prostate Score (GPS). In some embodiments, the quantitative score is used to predict relative risk of a clinical endpoint for a patient such as CR, BCR, Mets, and PCD. In some embodiments, the clinical endpoint is considered as of a particular time-period, such as 3 years, 5 years, or 10 years.

In some embodiments, the methods include determining whether the patient's GPS is above or below a particular threshold value suggestive of particular levels of risk for adverse clinical outcome or for various clinical endpoints such as CR, BCR, Mets, and PCD. In some embodiments, the threshold value is from 18-22, such as 18, 19, 20, 21, or 22 and in some embodiments it is from 38-42, such as 38, 39, 40, 41, or 42 and in some embodiments both of those threshold values are considered. In some embodiments, the threshold value is 20 and in some embodiments it is 40 and in some embodiments values of 20 and 40 are both considered.

As used herein, a "quantitative score" generally refers to an arithmetically or mathematically calculated numerical value for aiding in simplifying or disclosing or informing the analysis of more complex quantitative information, such as the correlation of certain expression levels of the disclosed genes or gene subsets to a likelihood of a particular clinical outcome parameter for a prostate cancer patient. A quantitative score may be determined by the application of a specific algorithm. In some embodiments herein, a quantitative score may be determined for a particular subset of genes or a gene group (i.e. a "gene group score"). The formation of groups, in addition, can facilitate the mathematical weighting of the contribution of various expression levels of genes or gene subsets to the quantitative score. The weighting of a gene or gene group representing a physiological process or component cellular characteristic can reflect the contribution of that process or characteristic to the pathology of the cancer and clinical outcome, such as CR, BCR, Mets, and PCD.

The GPS is calculated from expression level data for a set of genes comprising each of the following genes: BGN, COL1A1, SFRP4, FLNC, GSN, GSTM2, TPM2, TPX2, AZGP1, FAM13C, KLK2, and SRD5A2, and for at least one reference gene such as one or more of GUS, ARF1, ATP5E, CLTC, GPS1, and PGK1.

The analyzed genes may be placed into particular gene subsets as part of the algorithm. The gene subsets of the present disclosure include, for example, a stromal response gene group, a proliferation gene group, an androgen signaling gene group, and a cellular organization gene group. The stromal response and proliferation gene groups comprise genes associated with worse outcome when over-expressed whereas the androgen signaling and cellular organization gene groups comprise genes associated with worse outcomes when under-expressed.

The gene subset referred to herein as the and "stromal response gene group" (also called the "ECM gene group" or "stromal gene group") includes the BGN, COLIA1, and SFRP4 genes. Genes in this group may be synthesized predominantly by stromal cells and may be involved in stromal response or may co-express with the genes of the ECM gene group. "Stromal cells" are referred to herein as connective tissue cells that make up the support structure of biological tissues. Stromal cells include fibroblasts, immune cells, pericytes, endothelial cells, and inflammatory cells.

The "cellular organization gene group" (also called the "migration gene group" or "migration regulation gene group" or "cytoskeletal gene group") includes the FLNC, GSN, GSTM2, and TPM2 genes. These genes may comprise genes and co-expressed genes that are part of a dynamic microfilament network of actin and accessory proteins and that provide intracellular support to cells, generate the physical forces for cell movement and cell division, as well as facilitate intracellular transport of vesicles and cellular organelle.

The "androgen gene group" (also called the "PSA gene group," and "PSA regulation gene group") includes the AZGP1, FAM13C, KLK2, AR, ERG and SRD5A2 genes. These genes may include genes that are members of the kallikrein family of serine proteases (e.g. kallikrein 3 [PSA]), and genes that co-express with genes of the androgen gene group.

The "proliferation gene group" (also called the "cell cycle gene group") comprises the TPX2 gene. This gene group includes genes that may be involved with cell cycle functions such as cell proliferation and cell cycle control, e.g., checkpoint/G1 to S phase transition, and genes that co-express with such genes.

In some embodiments, an algorithm selected from the RS0 to RS27 algorithms of Table 5B of WO 2013/116144 may be selected and optionally scaled to between 0 and 100 to obtain a quantitative score from which to evaluate a patient, for instance, to determine relative risk of a clinical endpoint such as CR, BCR, Mets, or PCD. In some embodiments, the gene set comprises at least one gene from each of the stromal response group, the cellular organization group, the androgen group, and the proliferation group. In some embodiments, the algorithm may be modified, for example, to add or remove one or more genes from one or more of the gene groups discussed above, or to add a further gene group. In some embodiments, the clinical endpoint is considered as of a particular time-period, such as 3 years, 5 years, or 10 years.

**Calculation of GPS**

In some embodiments, the quantitative result is GPS. GPS result may be calculated on a scale from 0 to 100, and may be derived from reference-normalized gene expression measurements as follows.
Unscaled GPS (GPSu) may be calculated as:

$$\text{GPSu} = 0.735*\text{Stromal Response group score} - 0.368* \text{Cellular Organization group score} - 0.352*\text{Androgen group score} + 0.095*\text{Proliferation group score}$$

Where:

The Stromal Response group score = 0.527*BGN + 0.457*COL1A1 + 0.156*SFRP4
The Cellular Organization group score = 0.163*FLNC + 0.504*GSN + 0.421*TPM2 + 0.394*GSTM2
The Androgen group score = 0.634*FAM13C + 1.079*KLK2 + 0.642*AZGP1 + 0.997*SRD5A2 Thresh
The Proliferation group score = TPX2 Thresh
where the SRD5A2 Thresh and TPX2 Thresh are calculated via thresholding as follows:

$$\text{SRD5A2 Thresh} = \begin{cases} 5.5 & \text{if SRD5A2} < 5.5 \\ \text{SRD5A2} & \text{otherwise} \end{cases}$$

$$\text{TPX2 Thresh} = \begin{cases} 5.0 & \text{if TPX2} < 5.0 \\ \text{TPX2} & \text{otherwise} \end{cases}$$

GPSu may then be scaled to be between 0 and 100 as follows:

$$\text{GPS(scaled)} = \begin{cases} 0 & \text{if } 13.4 \times (\text{GPSu}+10.5) < 0 \\ 13.4 \times (\text{GPSu}+10.5) & \text{if } 0 \le 13.4 \times (\text{GPSu}+10.5) \le 100 \\ 100 & \text{if } 13.4 \times (\text{GPSu}+10.5) > 100 \end{cases}$$

The GPS(scaled) is the GPS value.
Once the GPS value or result is obtained for a patient, the score may then be classified into particular GPS groups using pre-specified cut-points or thresholds, such as cut-points in the range of 18-22, such as 18, 19, 20, 21, or 22 and/or in the range of 38-42, such as 38, 39, 40, 41, or 42. These cut-points may be defined based on statistical analyses of risks of CR, BCR, Mets, and PCD vs. GPS result, as described in the examples that follow.
In some embodiments, a patient may be placed into two groups based on a cut-point in the range of 18-22, such as 18, 19, 20, 21, or 22, where one group is considered below the cut-point if the GPS result is less than or alternatively less than or equal to the cut-point and the other group is considered above the cut-point if the GPS result is greater than or alternatively greater than or equal to the cut-point. Thus, for example, a low GPS group could be one with a GPS < 20 (or < 18, < 19, < 21, < 22, depending upon where the cut-point is set) and a high GPS group could be one with a GPS $\ge$ 20 (or $\ge$ 18, $\ge$ 19, $\ge$ 21, $\ge$ 22, depending upon where the cut-point is set). Or, alternatively, a low GPS group could be one with a GPS $\le$ 20 and a high GPS group could be one with a GPS > 20 (or $\le$ 18, $\le$ 19, $\le$ 21, $\le$ 22,

and > 18, > 19, > 21, > 22, depending on where the cut-point is set). In some embodiments, a cut-point of 20 defines a low GPS group and a higher GPS group such that those with GPS result $\leq 20$ fall in the low GPS group and those with GPS result > 20 fall in the higher GPS group.

In some embodiments, a cut point in the range of 38-42 is used to signify a low GPS and a high GPS group. Thus, for example, a low GPS group could be one with a GPS < 40 (or < 38, < 39, < 41, < 42, depending upon where the cut-point is set) and a high GPS group could be one with a GPS $\geq 40$ ($\geq 41$, $\geq 42$, depending upon where the cut-point is set). Or, alternatively, a low GPS group could be one with a GPS $\leq 40$ and a high GPS group could be one with a GPS > 40 (or $\leq 41$, $\leq 42$, and > 38, > 39, > 41, > 42, depending on where the cut-point is set). In some embodiments, a cut-point of 40 defines a low GPS group and a higher GPS group such that those with GPS result $\leq 40$ fall in the low GPS group and those with GPS result > 40 fall in the higher GPS group.

## APPLYING THE GPS ALGORITHM TO PARTICULAR PROSTATE CANCER RISK GROUPS

Treatment for prostate cancer patients may, in some cases, be based at least in part on whether the patient has been classified according to one or more of the AUA, NCCN, or CAPRA standards, as very low, low, intermediate, or high risk for negative clinical outcomes. As noted above, these classifications take into account multiple factors such as tumor stage or grade, PSA levels, and Gleason score.

For example, available treatments for prostate cancer patients include surgery, such as radical prostatectomy (RP), transurethral resection of the prostate (TURP), excision, dissection, and tumor biopsy/removal. Dissection of pelvic lymph nodes (PLND) may also be performed in conjunction with RP in some cases, such as where there is concern regarding preventing future metastasis. In some cases radiation therapy (RT) may be performed, such as external beam radiation therapy (EBRT), primary brachytherapy, or other types of brachytherapy. In some cases a patient may be treated with drugs, such as androgen deprivation agents (androgen deprivation therapy or ADT), which generally comprise LHRH antagonists. ADT may be given in some cases before, during, and/or after RT, for example. In high risk patients, for example, ADT may be given for an extended period such as for 1, 2, 3, or more years following RT. In some cases, immunotherapy and chemotherapy agents may also be prescribed, such as docetaxel, cabazitaxel, or sipuleucel-T. Lower risk patients may also be treated solely by active surveillance and may, for example, remain on active surveillance unless or until there is evidence of a change in their tumor status. Elderly patients or those for whom life expectancy is otherwise short may also be treated merely by watchful waiting with palliative interventions when needed.

Different treatment choices may be made according to the level of risk for the patient and the associated recommendations by, for example, AUA or NCCN guidelines. For example, very low risk patients may be treated by active surveillance or, if their life expectancy is short, by watchful waiting. Low risk patients may be treated, for example, by active surveillance, but also by RP with optional PLND particular if a localized tumor can be completely removed, or by RT such as EBRT or brachytherapy, or by some other form of "single modality" treatment (i.e. one form of treatment such as surgery or radiation as opposed to a combination of the two). Intermediate risk patients may be treated, for example, with RP and optional PLND, by RT with optional ADT, or by a combination of surgery, radiation therapy, and optional drug treatment. Such patients may be treated by "multimodal" therapy - i.e. combinations of different forms of treatment such as RT and ADT. High risk patients may be treated, for example, with multimodal therapy such as EBRT and long-term ADT treatment, such as for 1, 2, or 3 years following RT, and sometimes further with chemotherapy if the patient is fit enough to handle the treatment. High risk patients may also be surgically treated if conditions warrant.

In some embodiments of the present methods, therefore, the patient has previously been placed into a very low, low, intermediate, or high risk group according to the AUA or NCCN or CAPRA standards. In some emdodiments, obtaining a GPS result or other quantitative score from a method described herein may be useful in determining an appropriate treatment strategy for the patient.

For example, in some embodiments, a GPS result is obtained for a very low or low risk patient and the method comprises determining which GPS result group the patient falls into, e.g. a low or high group using a cut-off point between 18 and 22 or a cut-off point between 38 and 42, or both of those cut-off points. In some embodiments, a GPS result is obtained for a high risk patient and the method comprises determining which GPS result group the patient falls into, e.g. a low or high group using a cut-off point between 18 and 22 or a cut-off point between 38 and 42, or both of those cut-off points. In some embodiments, a GPS result is obtained for an intermediate risk patient and the method comprises determining which GPS result group the patient falls into, e.g. a low or high group using a cut-off point between 18 and 22 or a cut-off point between 38 and 42, or both of those cut-off points.

In some such embodiments involving an intermediate risk patient, determination of a quantitative score based on the methods herein may be used to further classify the patient's relative risk of a negative clinical outcome. For example, in some embodiments, an intermediate risk patient with a GPS result of greater than 38, 39, 40, 41, or 42 or greater than or equal to 38, 39, 40, 41, or 42 indicates that the patient has a relatively high risk of negative clinical endpoints such as CR, BCR, Mets and PCD compared to intermediate risk patients as a whole. In some embodiments, an intermediate risk patient with a GPS result of greater than 38, 39, 40, 41, or 42 or greater than or equal to 38, 39, 40, 41, or 42 indicates that the patient has a risk of negative clinical endpoints such as CR, BCR, Mets and PCD that is similar to that of high risk patients as a whole. For example, an intermediate risk patient with a GPS result of greater than 40 or greater than or equal to 40 may indicate that the patient has a risk of negative clinical endpoints such as CR, BCR, Mets and PCD that is similar to that of high risk patients as a whole. In some embodiments, this information may affect how that intermediate risk patient is treated. For example, such a patient may be treated according to the recommendations of treatment for high risk patients and may, for example, receive multimodal treatment such as RT combined with ADT and optionally chemotherapy or immunotherapy. On the other hand, for an intermediate risk patient, a GPS result of less than 38, 39, 40, 41, or 42 or less than or equal to 38, 39, 40, 41, or 42 may indicate that the patient has a lower relative risk of negative clinical endpoints such as CR, BCR, Mets and PCD compared to intermediate risk patients as a whole. In some embodiments, for an intermediate risk patient, a GPS result of less than 40 or less than or equal to 40 may indicate that the patient has a lower relative risk of negative clinical endpoints such as CR, BCR, Mets and PCD compared to intermediate risk patients as a whole. In such cases, the patient may be treated with active surveillance, at least initially, rather than with surgery, or may be treated with single modality treatment, such as surgery or radiation alone. In some embodiments, for example involving an intermediate risk patient, a GPS result of less than 18, 19, 20, 21, or 22, or less than or equal to 18, 19, 20, 21, or 22 indicates a relatively low risk of negative clinical endpoints such as CR, BCR, Mets and PCD. In some embodiments, for example involving an intermediate risk patient, a GPS result of less than 20 or less than or equal to 20 indicates a relatively low risk of negative clinical endpoints such as CR, BCR, Mets and PCD. In such cases, for example, the patient may be treated with active surveillance, at least initially, rather than with surgery or radiation. In some embodiments, an intermediate risk patient may have a GPS result that is in between these upper and lower cut points. In such cases, the patient may be treated with active surveillance, at least initially, rather than with surgery, or may be treated with single modality treatment, such as surgery or radiation alone. Thus, GPS may allow segregation of intermediate risk patients into two or three sub-groups of higher and lower risk for negative clinical endpoints. Accordingly, in some embodiments, following determination of the GPS result and analysis of the patient's grouping, a change of treatment strategy may follow. In other embodiments, an initial treatment strategy may be based at least in part on a combination of risk group (e.g., AUA, NCCN, or CAPRA) and GPS result.

## METHODS OF ASSAYING EXPRESSION LEVELS OF A GENE PRODUCT

Various technological approaches for determination of expression levels of the disclosed genes are set forth in this specification, including, without limitation, RT-PCR, microarrays, high-throughput sequencing, serial analysis of gene expression (SAGE) and Digital Gene Expression (DGE), which will be discussed in detail below. In particular aspects, the expression level of each gene may be determined in relation to various features of the expression products of the gene including exons, introns, protein epitopes and protein activity.

The expression product that is assayed can be, for example, RNA or a polypeptide. The expression product may be fragmented. For example, the assay may use primers that are complementary to target sequences of an expression product and could thus measure full transcripts as well as those fragmented expression products containing the target sequence. Further information is provided in Table A of International Patent Publication No. WO2013/116144.

The RNA expression product may be assayed directly or by detection of a cDNA product resulting from a PCR-based amplification method, e.g., quantitative reverse transcription polymerase chain reaction (qRT-PCR). (*See e.g.,* U.S. Patent No. 7,587,279). Polypeptide expression product may be assayed using immunohistochemistry (IHC) by proteomics techniques. Further, both RNA and polypeptide expression products may also be assayed using microarrays.

Methods of gene expression profiling include methods based on hybridization analysis of polynucleotides, methods based on sequencing of polynucleotides, and proteomics- based methods. Exemplary methods known in the art for the quantification of RNA expression in a sample include northern blotting and *in situ* hybridization (Parker & Barnes, Methods in Molecular Biology 106:247-283 (1999)); RNAse protection assays (Hod, Biotechniques 13:852-854 (1992)); and PCR-based methods, such as reverse transcription PCR (RT-PCR) (Weis et al., Trends in Genetics 8:263-264 (1992)). Antibodies may be employed that can recognize sequence-specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS). Other methods known in the art may be used.

## Reverse Transcription PCR (RT-PCR)

Typically, mRNA is isolated from a test sample. The starting material is typically total RNA isolated from a human tumor, usually from a primary tumor. Optionally, normal tissues from the same patient can be used as an internal control. Such normal tissue can be histologically-appearing normal tissue adjacent to a tumor. mRNA can be extracted from a tissue sample, e.g., from a sample that is fresh, frozen (e.g. fresh frozen), or paraffin-embedded and fixed (e.g. formalin-fixed).

General methods for mRNA extraction are known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using a purification kit, buffer set and protease from commercial manufacturers, such as Qiagen, according to the manufacturer's instructions. For example, total RNA from cells in culture can be isolated using Qiagen RNeasy® mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

The sample containing the RNA is then subjected to reverse transcription to produce cDNA from the RNA template, followed by exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

PCR-based methods use a thermostable DNA-dependent DNA polymerase, such as a Taq DNA polymerase. For example, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction product. A third oligonucleotide, or probe, can be designed to facilitate detection of a nucleotide sequence of the amplicon located between the hybridization sites the two PCR primers. The probe can be detectably labeled, e.g., with a reporter dye, and can further be provided with both a fluorescent dye, and a quencher fluorescent dye, as in a Taqman® probe configuration. Where a Taqman® probe is used, during the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, high-throughput platforms such as the ABI PRISM 7700 Sequence Detection System® (Perkin-Elmer-Applied Biosystems, Foster City, CA, USA), or LightCycler® (Roche Molecular Biochemicals, Mannheim, Germany). In a preferred embodiment, the procedure is run on a LightCycler® 480 (Roche Diagnostics) real-time PCR system, which is a microwell plate-based cycler platform.

5'-Nuclease assay data are commonly initially expressed as a threshold cycle ("$C_t$"). Fluorescence values are recorded during every cycle and represent the amount of product amplified to that point in the amplification reaction. The threshold cycle ($C_t$) is generally described as the point when the fluorescent signal is first recorded as statistically significant. Alternatively, data may be expressed as a crossing point ( "Cp"). The Cp value is calculated by determining the second derivatives of entire qPCR amplification curves and their maximum value. The Cp value represents the cycle at which the increase of fluorescence is highest and where the logarithmic phase of a PCR begins.

To minimize errors and the effect of sample-to-sample variation, RT-PCR is usually performed using an internal standard. The ideal internal standard gene (also referred to as a reference gene) is expressed at a quite constant level among cancerous and non-cancerous tissue of the same origin (i.e., a level that is not significantly different among normal and cancerous tissues), and is not significantly affected by the experimental treatment (i.e., does not exhibit a significant difference in expression level in the relevant tissue as a result of exposure to chemotherapy), and expressed at a quite constant level among the same tissue taken from different patients. For example, reference genes useful in the methods disclosed herein should not exhibit significantly different expression levels in cancerous prostate as compared to normal prostate tissue. Exemplary reference genes used for normalization comprise one or more of the following genes: GUS, AAMP, ARF1, ATP5E, CLTC, GPS1, and PGK1. Gene expression measurements can be normalized relative to the mean of one or more (e.g., 2, 3, 4, 5, or more) reference genes. Reference-normalized expression measurements can range from 2 to 15, where a one unit increase generally reflects a 2-fold increase in RNA quantity.

Real time PCR is compatible both with quantitative competitive PCR, where an internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

The steps of a representative protocol for use in the methods of the present disclosure use fixed, paraffin-embedded tissues as the RNA source. For example, mRNA isolation, purification, primer extension and amplification can be performed according to methods available in the art. (see, e.g., Godfrey et al. J. Molec. Diagnostics 2: 84-91 (2000); Specht et al., Am. J. Pathol. 158: 419-29 (2001)). Briefly, a representative process starts with cutting about 10

μm thick sections of paraffin-embedded tumor tissue samples. The RNA is then extracted, and protein and DNA depleted from the RNA-containing sample. After analysis of the RNA concentration, RNA is reverse transcribed using gene-specific primers followed by RT-PCR to provide for cDNA amplification products.

**Design of Intron-Based PCR Primers and Probes**

PCR primers and probes can be designed based upon exon or intron sequences present in the mRNA transcript of the gene of interest. Primer/probe design can be performed using publicly available software, such as the DNA BLAT software developed by Kent, W.J., Genome Res. 12(4):656-64 (2002), or by the BLAST software including its variations.

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. See S. Rrawetz, S. Misener, Bioinformatics Methods and Protocols: Methods in Molecular Biology, pp. 365-386 (Humana Press).

Other factors that can influence PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases, and exhibit Tm's between 50 and 80 0C, e.g. about 50 to 70 0C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press,. New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

Table A of International Patent Publication No. WO2013/116144 provides further information concerning primer, probe, and amplicon sequences that can be used with the genes disclosed herein.

**MassARRAY® System**

In MassARRAY-based methods, such as the exemplary method developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivarion of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

**Other PCR-based Methods**

Further PCR-based techniques that can find use in the methods disclosed herein include, for example, BeadArray® technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression® (BADGE), using the commercially available Luminex100 LabMAP® system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003).

**Microarrays**

Expression levels of a gene or microarray of interest can also be assessed using the microarray technique. In this method, polynucleotide sequences of interest (including cDNAs and oligonucleotides) are arrayed on a substrate. The arrayed sequences are then contacted under conditions suitable for specific hybridization with detectably labeled cDNA generated from RNA of a test sample. As in the RT-PCR method, the source of RNA typically is total RNA isolated from a tumor sample, and optionally from normal tissue of the same patient as an internal control or cell lines. RNA can be extracted, for example, from frozen or archived paraffin-embedded and fixed (e.g. formalin-fixed) tissue samples.

For example, PCR amplified inserts of cDNA clones of a gene to be assayed are applied to a substrate in a dense array. Usually at least 10,000 nucleotide sequences are applied to the substrate. For example, the microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After washing under stringent conditions to remove non-specifically bound probes, the chip is scanned by confocal laser microscopy or by another detection method, such as a CCD camera. Quantitation of hybridization of each arrayed element allows for assessment of corresponding RNA abundance.

With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et at, Proc. Natl. Acad. Sci. USA 93(2): 106-149 (1996)). Microarray analysis can be performed by commercially available equipment, following manufacturer's protocols, such as by using the Affymetrix GenChip® technology, or Incyte's microarray technology.

**Serial Analysis of Gene Expression (SAGE)**

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

**Gene Expression Analysis by Nucleic Acid Sequencing**

Nucleic acid sequencing technologies are suitable methods for analysis of gene expression. The principle underlying these methods is that the number of times a cDNA sequence is detected in a sample is directly related to the relative expression of the RNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9): 1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

**Isolating RNA from Body Fluids**

Methods of isolating RNA for expression analysis from blood, plasma and serum (see, e.g., K. Enders, et al., Clin Chem 48,1647-53 (2002) (and references cited therein) and from urine (see, e.g., R. Boom, et al., J Clin Microbiol. 28, 495-503 (1990) and references cited therein) have been described.

**Immunohistochemistry**

Immunohistochemistry methods are also suitable for detecting the expression levels of genes and applied to the method disclosed herein. Antibodies (e.g., monoclonal antibodies) that specifically bind a gene product of a gene of interest can be used in such methods. The antibodies can be detected by direct labeling of the antibodies themselves, for example, with radioactive labels, fluorescent labels, hapten' labels such as, biotin, or an enzyme such as horse radish peroxidase or alkaline phosphatase. Alternatively, unlabeled primary antibody can be used in conjunction with a labeled secondary antibody specific for the primary antibody. Immunohistochemistry protocols and kits are well known in the art and are commercially available.

**Proteomics**

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N- terminal sequencing, and (3) analysis of the data using bioinformatics.

**General Description of the mRNA Isolation, Purification and Amplification**

The steps of a representative protocol for profiling gene expression using fixed, paraffin-embedded tissues as the RNA source, including mRNA isolation, purification, primer extension and amplification are provided in various published journal articles. (See, e.g., T.E. Godfrey, et al,. J. Molec. Diagnostics 2: 84-91 (2000); K. Specht et al., Am. J. Pathol. 158: 419-29 (2001), M. Cronin, et al., Am J Pathol 164:35-42 (2004)). Briefly, a representative process starts with cutting a tissue sample section (e.g.about 10 $\mu$m thick sections of a paraffin-embedded tumor tissue sample). The RNA is then extracted, and protein and DNA are removed. After analysis of the RNA concentration, RNA repair is performed if desired. The sample can then be subjected to analysis, e.g., by reverse transcribed using gene specific promoters followed by RT-PCR.

**STATISTICAL ANALYSES**

One skilled in the art will recognize that there are many statistical methods that may be used to determine whether there is a significant relationship between a clinical outcome of interest (e.g., recurrence) and GPS or another diagnostic test score.

For example, hypothesis tests can be reported using two-sided p-values. To investigate if there is a significant relationship of outcomes (eg. CR, BCR, Mets, PCD) with particular measured or calculated entities, Cox Proportional Hazards (PH) models using maximum weighted pseudo partial-likelihood estimators can be used and p-values from Wald tests of the null hypothesis that the hazard ratio (HR) is one can be reported.

**NORMALIZATION OF EXPRESSION LEVELS**

The expression data used in the methods disclosed herein can be normalized. Normalization refers to a process to correct for (i.e., normalize away), for example, differences in the amount of RNA and variability in the quality of the RNA obtained from a sample, to remove unwanted sources of systematic variation in Ct or Cp measurements, and the like. With respect to RT-PCR experiments involving archived fixed paraffin embedded tissue samples, for example, sources of systematic variation can include the degree of RNA degradation relative to the age of the patient sample and the type of fixative used to store the sample. Other sources of systematic variation are attributable to laboratory processing conditions.

Assays can provide for normalization by incorporating the expression of certain reference genes, which do not significantly differ in expression levels under the relevant conditions. Exemplary reference genes disclosed herein include housekeeping genes. (See, e.g., E. Eisenberg, et al., Trends in Genetics 19(7):362-365 (2003).) In general, the reference genes, are typically genes that are known not to exhibit meaningfully different expression in prostate cancer as compared to non-cancerous prostate tissue, and track with various sample and process conditions, thus provide for normalizing away extraneous effects. In exemplary embodiments, one or more of the following genes are used as reference genes by which mRNA expression data is normalized: GUS, AAMP, ARF1, ATP5E, CLTC, GPS1, and PGK1. The calibrated weighted average $C_T$ or Cp measurements for each of the test genes such as BGN, COL1A1, SFRP4, TPX2, AZGP1, FAM13C, KLK2, SRD5A2, FLNC, GSN, GSTM2, and TPM2, may be normalized relative to the mean of five or more reference genes. Normalization can, in other embodiments, alternatively be based on the mean or median signal (Ct or Cp) of all of the assayed genes or a large subset thereof (global normalization approach).

Those skilled in the art will recognize that normalization may be achieved in numerous ways, and the techniques described above are intended only to be exemplary, not exhaustive.

**STANDARDIZATION OF EXPRESSION LEVELS**

The expression data used in the methods disclosed herein can be standardized. Standardization refers to a process to effectively put all the genes on a comparable scale. This is performed because some genes will exhibit more variation (a broader range of expression) than others. Standardization is performed by dividing each expression value by its standard deviation across all samples for that gene. Hazard ratios are then interpreted as the proportional change in the hazard for the clinical endpoint (clinical recurrence, biological recurrence, death due to prostate cancer, or death due to any cause) per 1 standard deviation increase in expression.

## KITS OF THE INVENTION

The materials for use in the methods of the present invention are suited for preparation of kits produced in accordance with well-known procedures. The present disclosure thus provides kits comprising agents, which may include gene-specific or gene-selective probes and/or primers, for quantifying the expression of the disclosed genes for predicting prognostic outcome or response to treatment. Such kits may optionally contain reagents for the extraction of RNA from tumor samples, in particular fixed paraffin-embedded tissue samples and/or reagents for RNA amplification. In addition, the kits may optionally comprise the reagent(s) with an identifying description or label or instructions relating to their use in the methods of the present invention. The kits may comprise containers (including microliter plates suitable for use in an automated implementation of the method), each with one or more of the various materials or reagents (typically in concentrated form) utilized in the methods, including, for example, chromatographic columns, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present invention (e.g., appropriate length poly(T) or random primers linked to a promoter reactive with the RNA polymerase). Mathematical algorithms used to estimate or quantify prognostic or predictive information are also properly potential components of kits.

In some embodiments, a kit may comprise reagents necessary to determine levels of particular RNA transcripts in a patient sample by RT-PCR. For example, in some embodiments, a kit may comprise a cartridge or other similar physical structure comprising at least one well (which may constitute a channel, chamber, area, or surface) comprising one or more primers for determining levels of RNA transcripts of one or more of the BGN, COL1A1, SFRP4, TPX2, AZGP1, FAM13C, KLK2, SRD5A2, FLNC, GSN, GSTM2, and TPM2 genes. In some embodiments, the primers are attached to one or more wells in the cartridge. In some embodiments, each well may comprise primers for two, three, four, five, or six different genes, for example, by using primers labeled with different color labels. In some embodiments, at least one well comprises at least one primer for determining RNA transcript levels of one or more reference genes. In some embodiments, the reference gene comprises the GUS gene. In other embodiments, the reference gene comprises one or more of GUS, AAMP, ARF1, ATP5E, CLTC, GPS1, and PGK1. In some embodiments, the primers contained in the cartridge comprise primers for determining levels of RNA transcripts of a set of genes consisting of BGN, COL1A1, SFRP4, TPX2, AZGP1, FAM13C, KLK2, SRD5A2, FLNC, GSN, GSTM2, and TPM2 and of one or more reference genes. In some embodiments, the cartridge further comprises amplification reagents such as buffers, nucleotide triphosphates (e.g., dATP, dCTP, dGTP and dTTP; or rATP, rCTP, rGTP and UTP), reverse transcriptase, DNA polymerase, and/or RNA polymerase. In some embodiments, the cartridge is part of a system comprising one or more components that introduce these reagents into the wells of the cartridge. In some embodiments, RNA is extracted from the sample and the extracted RNA is applied to the cartridge. In other embodiments, no extraction step is needed and the sample is directly applied to the cartridge. Sample cartridges and associated systems that may be utilized to determine RNA transcript levels herein are described in International Publication No. WO2006/136990 and its associated US patent, No. 9,568,424.

In some embodiments, the cartridge comprises at least one well comprising primers where thermocycling takes place as well as one or more wells for introducing, lysing and/or washing the sample. In some embodiments, the overall cartridge structure also comprises pumps, valves, process wells, and fluid and waste resevoirs, which allows for conducting sample treatment and RT-PCR reactions and associated detection of RNA transcript levels of particular genes in the cartridge.

In some embodiments utilizing a cartridge system, the system is capable of determining RNA transcript levels using the RNA from the sample and the primers and reagents comprised in the cartridge, and the system also includes software capable of determining the associated normalized RNA transcript levels and calculating any associated quantitative scores, such as a GPS score. In some embodiments, the system is further able to determine whether the patient is at low, intermediate, or high risk of adverse clinical outcome, such as risk of clinical recurrence (CR), biochemical recurrence (BCR), distant metastasis (Mets), and prostate cancer death (PCD), by placing the patient's quantitative score (e.g., GPS score) into the appropriate low, intermediate, or high risk range as described herein. In some embodiments, the system is also capable of creating a report providing a patient's quantitative score.

## REPORTS

The methods of this invention, when practiced for commercial diagnostic purposes, generally produce a report or summary of information obtained from the herein-described methods. For example, a report may include information concerning expression levels of one or more genes, GPS result, comparison of the GPS result to particular threshold or cut-off points and/or to the mean score for prostate patients, as well as other information about the patient such as AUA or NCCN or other recognized risk group, and information used to place the patient into such a risk group such as PSA level, Gleason score, etc. The methods and reports of this invention can further include storing the report in a database. The method can create a record in a database for the subject and populate the record with data. The report may be a paper report, an auditory report, or an electronic record. The report may be displayed and/or stored on a computing device (e.g., handheld device, desktop computer, smart device, website, etc.). It is contemplated that the report is provided to a physician and/or the patient. The receiving of the report can further include establishing a network connection to a server computer that includes the data and report and requesting the data and report from the server computer.

## COMPUTER PROGRAM

The values from the assays described above, such as expression data, can be calculated and stored manually. Alternatively, the above-described steps can be completely or partially performed by a computer program product. The present invention thus provides a computer program product including a computer readable storage medium having a computer program stored on it. The program can, when read by a computer, execute relevant calculations based on values obtained from analysis of one or more biological samples from an individual (e.g., gene expression levels, normalization, standardization, thresholding, and conversion of values from assays to a score and/or text or graphical depiction of tumor stage and related information). The computer program product has stored therein a computer program for performing the calculation.

The present disclosure provides systems for executing the program described above, which system generally includes: a) a central computing environment; b) an input device, operatively connected to the computing environment, to receive patient data, wherein the patient data can include, for example, expression level or other value obtained from an assay using a biological sample from the patient, or microarray data, as described in detail above; c) an output device, connected to the computing environment, to provide information to a user (e.g., medical personnel); and d) an algorithm executed by the central computing environment (e.g., a processor), where the algorithm is executed based on the data received by the input device, and wherein the algorithm calculates an expression score, thresholding, or other functions described herein. The methods provided by the present invention may also be automated in whole or in part.

Having described the invention, the same will be more readily understood through reference to the following Examples, which are provided by way of illustration, and are not intended to limit the invention in any way.

## EXAMPLES

**Example 1: Risk of Clinical Recurrence (CR) and Prostate Cancer Death (PCD) Associated With a GPS result < 20**

Two large longitudinal prostate cancer cohorts were analyzed to estimate the risk of CR and PCD for GPS < or > 20 units on a scale of 0 to 100. Patient data from E. Klein et al., Eur Urol 66: 550-560 (2014) and J. Cullen, et al., Eur Urol 68: 123-131 (2015) were analyzed to establish the risk of CR ad PCD associated with a pre-established GPS cut-off point of 20. See E. Klein et al., Eur Urol 66: 550-560 (2014), Table 1, and J. Cullen, et al., Eur Urol 68: 123-131 (2015), Table 1, for further details regarding the baseline characteristics of the patients in this study.

Patients were divided based on the value of GPS (either $\leq$ 20 or > 20). Cox regression analyses accounted for cohort sampling weights. Since GPS was developed using Klein's standardized hazard ratios (std HR, HR for 1 standard deviation (SD) change in the covariate) for GPS and CR and PCD survival curves for the 2 groups were estimated correcting for regression to the mean (RM).

Of the 402 patients in Cullen (median follow up 5.2 years), only 5 patients developed metastases and all 5 had GPS > 20. Of the 426 patients in Klein with a median follow up of 6.6 years, there were 109 CR (including both distant metastasis and local recurrences) and 39 PCD, but only one such patient had a GPS < 20. Comparatively, 28% of patients from Klein had GPS < 20. GPS was a significant predictor for both CR (std HR 2.50 (95% CI 1.99, 3.15, p < 0.001, RM-corrected std HR 2.16, FDR < 0.1%) and PCD (std HR 2.90 (95%CI 2.06, 4.06, p < 0.001, RM-corrected std HR 1.96, FDR < 0.1%) after adjustment for AUA risk group. As shown in the table below, men with intermediate risk prostate cancer (AUA) and a GPS result of $\leq$ 20 have a 2.6% and 0.7% 10-year RM-corrected risk of CR and PCD, respectively. Men with an intermediate risk prostate cancer (AUA) and a GPS result of > 20 have greater estimated 10-year RM-corrected risks of CR and PCD. These results suggest that men in the NCCN very low, low, or intermediate risk groups and a GPS result $\leq$ 20 may be suitable candidates for active surveillance rather than immediate definitive treatment.

**Table 1**

| Estimated 10-year RM-corrected risk of CR and PCD | | | |
|---|---|---|---|
| AUA Risk Group | GPS Group | CR Risk | PCD Risk |
| Low | ≤ 20 | 1.8% | 0.5% |
| | > 20 | 4.3% | 1.0% |
| Intermediate | ≤ 20 | 2.6% | 0.7% |
| | > 20 | 10.9% | 3.1% |
| High | ≤ 20 | 6.0% | 2.1% |
| | > 20 | 21.2% | 7.8% |

**Example 2: GPS results of ≤ 40 and > 40 and Risk of Distant Metastasis and Prostate Cancer Death in Prostate Cancer Patients**

An initial selection of 259 patients were chosen from a 1995-2010 large, community-based U.S. Integrated health care system of 6184 prostate cancer patients with NCCN risks from very low to high. Specifically, among 6,184 eligible patients, 404 were selected based on a pre-specified cohort sampling schema, of which 334 patients had available biopsy tissues. There were 14 (4%) excluded because of clinical ineligibility, 41 (12%) due to insufficient tumor or incorrect tumor type. Of the remaining 279, valid GPS results were obtained for 259 (93%) patients, representing the final evaluable population The 259 patients included 5 in the very low risk group, 35 in the low risk group, 160 in the intermediate risk group, and 57 in the high risk group. The table below provides characteristics of the 259 evaluable patients.

**Table 2**

| Characteristic | Values | N (Weighted %) |
|---|---|---|
| Race/Ethnicity | White Non-hispanic | 201 (79.0) |
| | African-American | 26 (11.0) |
| | Other | 32 (10.0) |
| | | |
| PSA ng/ml | 0-4 | 24 (9.5) |
| | 4.1-10 | 159 (70.1) |
| | 10.1 and above | 75 (20.4) |
| | | |
| Clinical T-stage | T1 | 67 (24.9) |
| | T2 | 189 (74.6) |
| | T3 | 2 (0.4) |
| | | |
| Biopsy Gleason Score (central) | 3+3 | 69 (37.6) |
| | 3+4 | 113 (45.5) |
| | 4+3 | 42 (11.4) |
| | 4+4 | 12 (2.7) |
| | Any pattern 5 | 23 (2.8) |
| | | |
| NCCN Risk Group | Very Low | 5 (3.0) |
| | Low | 35 (20.6) |

(continued)

| Characteristic | Values | N (Weighted %) |
|---|---|---|
|  | Intermediate | 160 (67.1) |
|  | High | 57 (9.3) |

The 259 evaluable patients included 79 (weighted proportion=8.8%) distant metastases, and 180 non-metastases. The 259 evaluable patients included 64 PCD (weighted proportion=1.8%) and 195 non-PCD.

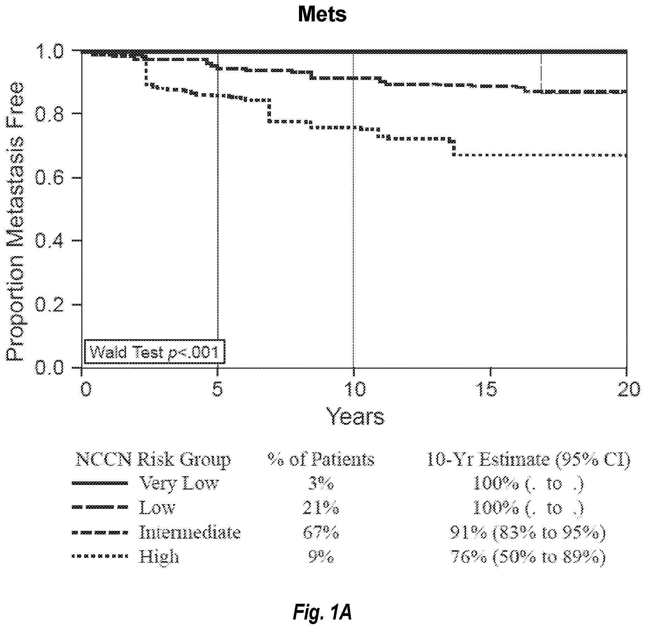

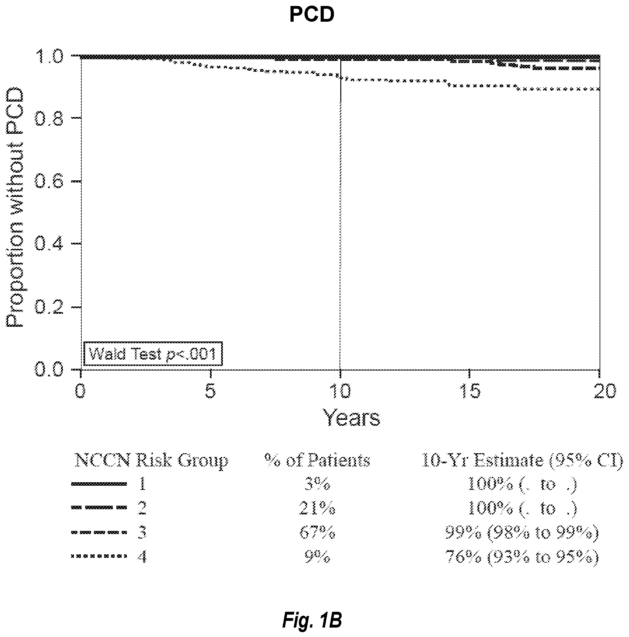

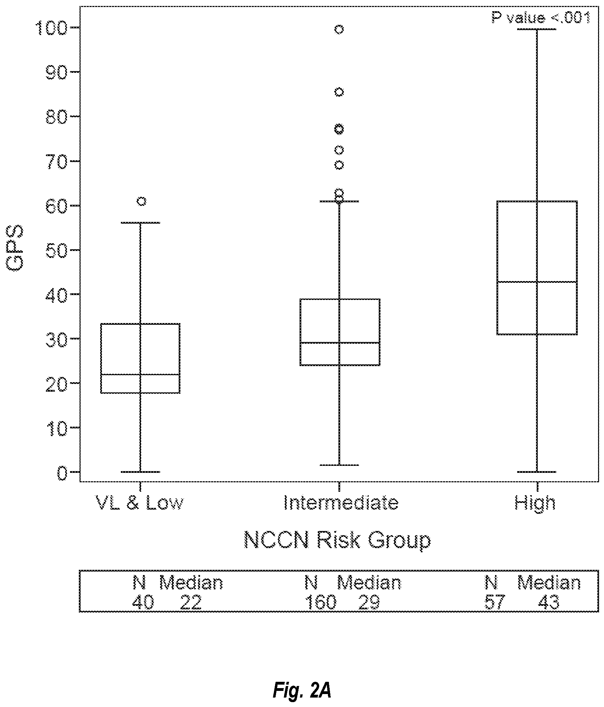

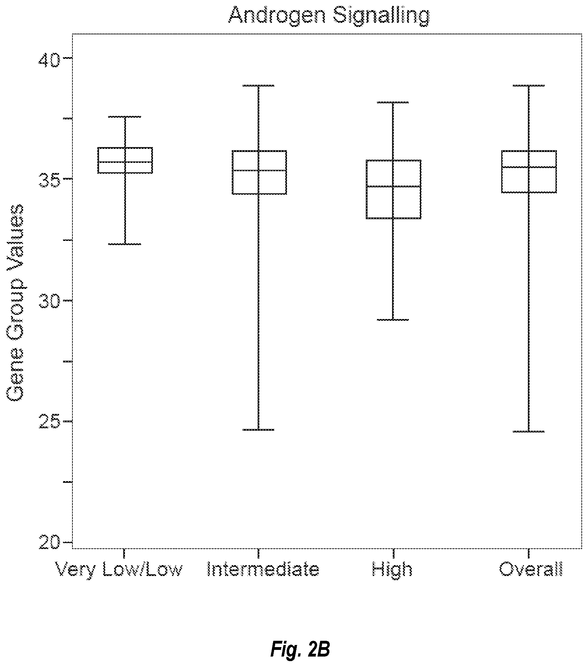

Ten-year estimates of distant metastases (Mets) and prostate cancer death (PCD) were determined for each of the patient risk groups and GPS results were obtained. (See Figs. 1A-1B and 2A-2B for details.) The mean GPS for all 259 patients was 31 and the median score was 28, as shown in the table below.

**Table 3**

|  | GPS |
|---|---|
| Mean | 31.3 |
| SD | 14.1 |
| Min | 0 |
| Q1 | 21.3 |
| Median | 28.4 |
| Q3 | 38.8 |
| Max | 100 |

For the very low and low risk group of 40 patients, the median score was 22, for the intermediate group of 160 patients, the median was 29, and for the high risk group of 57 patients, the median was 43.

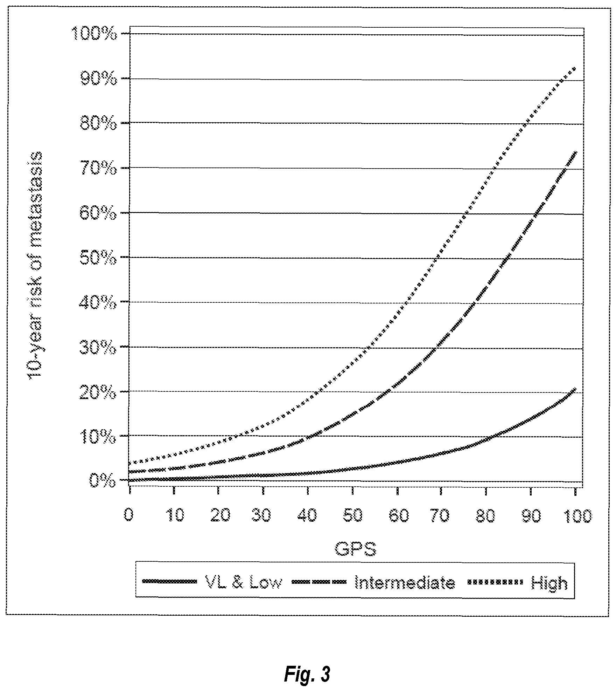

In a multivariate analysis (MVA) considering GPS against Gleason score, NCCN risk group, AUA risk group, or Capra score, GPS was found to be significantly associated with 10-year risk of Mets and PCD with p values from < 0.001 to 0.007. See the tables below for details. See also Fig. 3.

**Table 4: Association of GPS + Clinical Factors with Mets, *GPS is significant in all MVA***

| Model | Variable | N | HR | 95% CI | P-value |
|---|---|---|---|---|---|
| 1 | GPS | 259 | 2.01 | (1.21-3.33) | 0.007 |
|  | Bx Gleason Total |  |  |  | **0.004** |
|  | Bx Gleason Score 7 vs <=6 |  | 7.26 | (1.60-33.03) | 0.010 |
|  | Bx Gleason Score >=8 vs <=6 |  | 19.29 | (3.36-110.64) | <.001 |
|  |  |  |  |  |  |
| 2 | GPS | 257 | 2.34 | (1.42-3.86) | <.001 |
|  | NCCN Risk Group |  |  |  | 0.064 |
|  | NCCN High vs Very Low & Low |  | 11.02 | (1.44-84.29) | **0.021** |
|  | NCCN Int. vs Very Low & Low |  | 5.70 | (0.83-39.05) | 0.076 |
|  |  |  |  |  |  |
| 3 | GPS | 257 | 2.51 | (1.49-4.23) | <.001 |
|  | AUA Risk Group |  |  |  | 0.109 |
|  | AUA Intermediate vs Low |  | 5.53 | (0.75-40.73) | 0.093 |
|  | AUA High vs Low |  | 7.83 | (1.14-53.65) | 0.036 |
|  |  |  |  |  |  |

(continued)

| Model | Variable | | N | HR | 95% CI | P-value |
|---|---|---|---|---|---|---|
| 4 | GPS | | 257 | 2.63 | (1.58-4.36) | <u><.001</u> |
| | Capra | Score | | 1.23 | (1.00-1.52) | **0.050** |

**Table 5: Association of GPS with PCD in multivariable model, *GPS is significant in addition to clinical nomograms***

| Model | Variable | N | HR | 95% CI | P-value |
|---|---|---|---|---|---|
| 1 | GPS | 257 | 2.69 | (1.50-4.82) | <u><.001</u> |
| | NCCN Risk Group | | | | **0.017** |
| | NCCN High vs Very Low & Low | | 22.54 | (2.38-213.07) | 0.007 |
| | NCCN Int. vs Very Low & Low | | 8.59 | (1.06-69.56) | 0.044 |
| | | | | | |
| 2 | GPS | 257 | 3.04 | (1.79-5.18) | <u><.001</u> |
| | AUA Risk Group | | | | **0.013** |
| | AUA Int. vs Low | | 7.12 | (0.83-61.40) | 0.074 |
| | AUA High vs Low | | 16.79 | (1.99-141.78) | 0.010 |
| | | | | | |
| 3 | GPS | 257 | 3.40 | (2.04-5.64) | <u><.001</u> |
| | Capra Score | | 1.76 | (1.37-2.26) | **<.001** |

GPS result also remained significantly associated with PCD (p value = 0.004) in a multivariate model adjusting for clinical factors such as age at diagnosis, Gleason score, PSA level at diagnosis, and percent of biopsy cores positive. See the table below for details.

**Table 6: Association of GPS with PCD in multivariable model**

| Variable | N | HR | HR 95% CI | Wald P-value |
|---|---|---|---|---|
| GPS per 20 Units | 242 | 2.52 | (1.34, 4.76) | 0.004 |
| Age at Diagnosis | 242 | 1.07 | (0.99, 1.16) | 0.069 |
| Central Gleason Score 7 vs <=6 | 242 | 3.02 | (0.74, 12.29) | 0.122 |
| 8+ vs <=6 | 242 | 7.89 | (1.35, 46.24) | 0.022 |
| Percent of Biopsy Cores Positive | 242 | 11.47 | (1.89, 69.48) | 0.008 |
| PSA at diagnosis | 242 | 1.01 | (1.004, 1.02) | 0.003 |

In addition, analysis of the data from the 160 NCCN intermediate risk patients showed that patients with a GPS result of > 40 (24%) had a 5-year risk of metastases similar to that of high risk patients. Specifically, 84% of such patients were metastasis-free at 5 years, whereas 85% of high risk patients regardless of GPS result were metastasis-free at 5 years, and whereas 97% of intermediate risk patients with a GPS result of $\leq$ 40 were metastasis-free at 5 years. In addition, NCCN high risk patients with a GPS $\leq$ 40 (41% of the high risk group studied) had a 5-year risk of distant metastases similar to that of clinically intermediate risk patients. Specifically, those patients were estimated to be 96% metastasis-free at 5 years, while all intermediate patients were 94% metastasis-free at 5 years. In contrast, high risk patients with GPS > 40 were only 59% metastasis-free at 5 years.

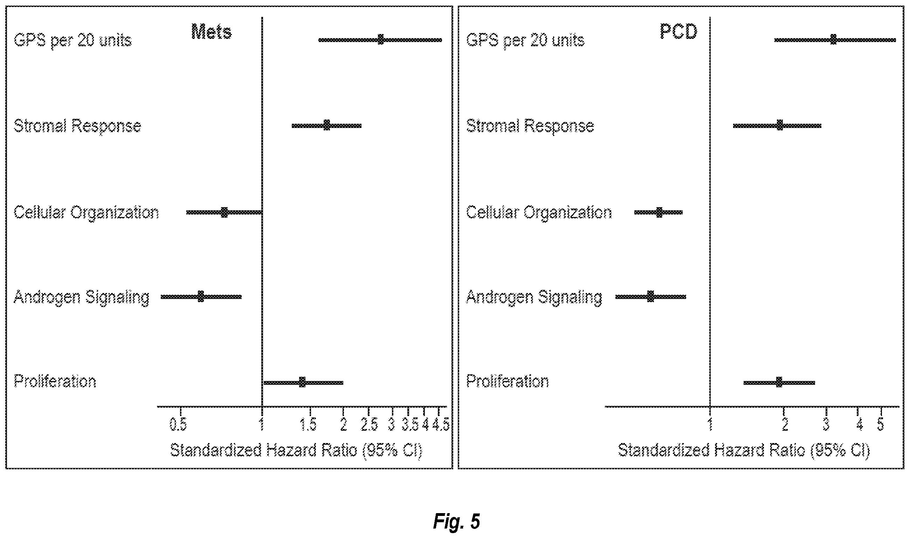

The individual gene group scores (stromal response, cellular organization, androgen signaling, and proliferation) were also determined and analyzed against occurrence of Mets and PCD. See Fig. 5. of the gene group scores with both Mets and PCD remains as expected based on associations of the gene group scores with other variables such as CR.

Overall, the results demonstrate that GPS is a significant predictor of risk of metastasis and PCD after radical prostatectomy for clinically low, intermediate, and high risk prostate cancer patients, that association between GPS and time to metastasis and PCD remains significant after adjusting for clinical and pathologic covariates including NCCN, AUA, and CAPRA clinical risk groups, and that GPS adds prognostic information beyond conventional clinicopathologic prognostic factors and improves risk stratification at the time of diagnosis. Furthermore, within the NCCN intermediate risk group, GPS was a significant predictor of outcomes. In particular, those with a GPS result above 40 had a 5-year risk of distant metastases similar to that of clinically high risk patients. As a result, these intermediate risk group patients with GPS > 40 may be suitable candidates for more intensified treatment such as multi-modality treatment.

The correlation between GPS and biochemical recurrence (BCR) was also studied in this patient group. For purposes of the study, a BCR event after surgery was defined according to the 2007 AUA guideline as (1) a post-surgery PSA level of $\geq$ 0.2 ng/mL with a successive confirmatory PSA level of > 0.2 ng/mL, where the BCR date is the first PSA date; or (2) the initiation of salvage radiation or hormonal therapy after a rising PSA level $\geq$ 0.1 ng/mL, where the BCR date is the salvage therapy date. The study found a significant association between GPS value per 20 units and BCR (HR 2.50; HR 95% CI 1.62, 3.85, Wald Chisq 17.00; Wald p-value < 0.0001) in the 259 studied patients in a univariate model.

**Table 7: Association of Clinical Factors with BCR**

| Model | Variable | N | HR | 95% CI | P-value |
|---|---|---|---|---|---|
| 1 | Age at Diagnosis | 259 | 1.00 | (0.95-1.05) | 0.949 |
| | | | | | |
| 2 | Race | 259 | | | 0.617 |
| | Black vs. White | | 1.56 | (0.64-3.83) | 0.330 |
| | Other vs. White | | 1.13 | (0.44-2.90) | 0.794 |
| | | | | | |
| 3 | Clinical Tstage | 258 | | | **<.001** |
| | cT-stage 2 vs 1 | | 0.88 | (0.45-1.71) | 0.700 |
| | cT-stage 3 vs 1 | | 17.45 | (8.34-36.52) | <.001 |
| | | | | | |
| 4 | Bx Gleason Total | 259 | | | **<.001** |
| | Bx Gleason Score 7 vs <=6 | | 3.79 | (1.58-9.09) | 0.003 |
| | Bx Gleason Score >=8 vs <=6 | | 12.66 | (4.85-33.03) | <.001 |
| | | | | | |
| 5 | % Positive Core Bx | 243 | 2.68 | (0.73-9.79) | 0.137 |
| | | | | | |
| 6 | Bx PSA Category | 258 | | | **0.014** |
| | PSA <4 vs 4 to <10 | | 1.80 | (0.67-4.82) | 0.242 |
| | PSA >=10 vs 4 to <10 | | 2.56 | (1.35-4.85) | 0.004 |
| | | | | | |
| 7 | PSA Density by 0.1 units | 243 | 1.16 | (1.05-1.28) | **0.003** |
| | | | | | |
| 8 | NCCN Risk Group | 257 | | | **<.001** |
| | NCCN Intermediate vs VL & Low | | 2.13 | (0.79-5.77) | 0.137 |
| | NCCN High vs VL & Low | | 9.91 | (3.60-27.32) | <.001 |
| | | | | | |

(continued)

| Model | Variable | N | HR | 95% CI | P-value |
|-------|----------|---|-----|--------|---------|
| 9 | AUA Risk Group | 257 | | | **0.025** |
| | AUA Intermediate vs Low | | 2.19 | (0.78-6.11) | 0.135 |
| | AUA High vs Low | | 3.72 | (1.35-10.23) | 0.011 |
| | | | | | |
| 10 | Capra Score | 257 | 1.69 | (1.42-2.01) | **<.001** |

**Table 8: Association of GPS with BCR in multivariable model** *GPS is significant in addition to clinical nomograms*

| Model | Variable | N | HR | 95% CI | P-value |
|-------|----------|---|-----|--------|---------|
| 1 | GPS | 257 | 2.11 | (1.41-3.14) | <.001 |
| | NCCN Risk Group | | | | **0.001** |
| | NCCN High vs Very Low & Low | | 5.21 | (1.84-14.79) | 0.002 |
| | NCCN Int. vs Very Low & Low | | 1.68 | (0.61-4.65) | 0.318 |
| | | | | | |
| 2 | GPS | 257 | 2.41 | (1.64-3.54) | <.001 |
| | AUA Risk Group | | | | 0.061 |
| | AUA Int. vs Low | | 1.53 | (0.52-4.45) | 0.439 |
| | AUA High vs Low | | 2.74 | (0.97-7.75) | 0.058 |
| | | | | | |
| 3 | GPS | 257 | 2.30 | (1.58-3.36) | <.001 |
| | Capra Score | | 1.56 | (1.31-1.85) | **<.001** |

**Table 9: Association of GPS with BCR in multivariable model**

| Variable | N | HR | HR 95% CI | Wald P-value |
|----------|---|-----|-----------|--------------|
| GPS per 20 Units | 258 | 2.07 | (1.32. 3.25) | 0.002 |
| cT-stage T2 vs T1 | 258 | 0.79 | (0.40, 1.56) | 0.499 |
| T3 vs T1 | | 9.08 | (4.03, 20.45) | <.0001 |
| Central Gleason Score 7 vs <=6 | 258 | 2.67 | (1.12, 6.37) | 0.027 |
| 8+ vs <=6 | | 5.32 | (1.94, 14.56) | 0.001 |
| PSA at diagnosis | 258 | 1.02 | (1.01, 1.02) | <.0001 |

The association remained significant (p < 0.001) in a multivariate model considering NCCN, AUA, and Capra scores as well as GPS result. See Table 4 for details. The association also remained significant after adjusting for factors such as cT stage, Gleason score, and PSA at diagnosis. See Table 5 for details.

**Example 3: GPS results of ≤ 40 and > 40 and Risk of Clinical Recurrence (CR) and Biochemical Recurrence (BCR) in Prostate Cancer Patients**

In two further studies, patient data from E. Klein et al., Eur Urol 66: 550-560 (2014) and J. Cullen, et al., Eur Urol 68: 123-131 (2015) were analyzed to consider how a GPS result above or below 40 correlates with BCR and CR in intermediate risk patients. See also E. Klein et al., Eur Urol 66: 550-560 (2014), Table 1, and J. Cullen, et al., Eur Urol 68: 123-131 (2015), Table 1, for further details regarding the baseline characteristics of the patients in these studies.

A study of prostate cancer patients from a Cleveland Clinic (CC) database described in E. Klein et al., Eur Urol 66: 550-560 (2014) showed that AUA intermediate risk group patients with GPS ≤ 40 had an RM-corrected estimated risk of clinical recurrence (CR) by 10 years of 4.7%, whereas AUA intermediate risk patients with GPS > 40 had an RM-corrected estimated risk of CR by 10 years of 16.9%. Patients in the AUA high risk group had an RM-corrected estimated risk of CR by 10 years of 18.2% regardless of GPS result. Thus, the high GPS intermediate and high risk groups had similar risks of CR by 10 years.

Patients in the AUA intermediate risk group with GPS ≤ 40 were also found to have an RM-corrected estimated risk of biochemical recurrence (BCR) (PSA threshold of 0.2) by 3 years of 15.7% and by 5 years of 23.6%, whereas AUA intermediate risk patients with GPS > 40 were found to have an RM-corrected estimated risk of BCR by 3 years of 33.5% and by 5 years of 47.1%. Patients in the AUA high risk group had an RM-corrected estimated risk of BCR by 3 years of 32.9% and by 5 years of 45.4% regardless of GPS result. Again, the high GPS intermediate and high risk groups had similar risks of BCR by 3 and 5 years.

In a similar analysis of patients from a Center for Prostate Disease Research database (CPDR) study described in J. Cullen, et al., Eur Urol 68: 123-131 (2015), a group of 139 NCCN intermediate risk patients was evaluated to consider GPS result vs 3-year or 5-year risk of BCR. Intermediate risk patients with a GPS of ≤ 40 (61% of the patients) had a 3-year risk of BCR (PSA threshold of 0.2) of 8.0% and a 5-year risk of BCR of 16.1%. In contrast, those with a GPS > 40 had a 3-year risk of BCR of 27.4% and a 5-year risk of BCR of 36.0%. These results are similar to those for the patients in the CC/Klein study.

**Claims**

1. A method of assigning a relative risk of adverse clinical outcome to a prostate cancer patient, comprising:

   (a) measuring, in a biological sample containing cancer cells obtained from the patient, levels of RNA transcripts of the following genes: BGN, COL1A1, SFRP4, FLNC, GSN, TPM2, GSTM2, FAM13C, KLK2, AZGP1, SRD5A2, and TPX2;

   (b) normalizing the levels of the RNA transcripts of the genes to obtain normalized gene expression levels;

   (c) calculating a quantitative score (QS) for the patient, wherein the quantitative score is calculated as follows, wherein the gene symbols below represent the normalized gene expression levels for each respective gene:

   (i) calculating an unscaled quantitative score (QSu) as follows:

$$QSu = 0.735*\text{Stromal Response group score} -0.368* \text{ Cellular Organization group score} -0.352*\text{Androgen group score} + 0.095*\text{Proliferation group score}$$

   Where:

   The Stromal Response group score = 0.527*BGN + 0.457*COL1A1 + 0.156*SFRP4
   The Cellular Organization group score = 0.163*FLNC + 0.504*GSN + 0.421*TPM2 + 0.394*GSTM2
   The Androgen group score = 0.634*FAM13C + 1.079*KLK2 + 0.642*AZGP1 + 0.997*SRD5A2 Thresh
   The Proliferation group score = TPX2 Thresh

   where the SRD5A2 Thresh and TPX2 Thresh are calculated via thresholding as follows:

$$SRD5A2 \text{ Thresh} = \begin{cases} 5.5 & \text{if SRD5A2} < 5.5 \\ SRD5A2 & \text{otherwise} \end{cases}$$

$$\text{TPX2 Thresh} = \begin{cases} 5.0 & \text{if TPX2} < 5.0 \\ \text{TPX2} & \text{otherwise} \end{cases}$$

(ii) calculating a scaled quantitative score (QS) where:

$$\text{QS(scaled)} \begin{cases} 0 & \text{if } 13.4 \times (\text{QSu}+10.5) < 0 \\ 13.4 \times (\text{QSu}+10.5) & \text{if } 0 \leq 13.4 \times (\text{QSu}+10.5) \leq 100 \\ 100 & \text{if } 13.4 \times (\text{QSu}+10.5) > 100 \end{cases}$$

(d) assigning the patient to a quantitative score group, wherein (i) the patient is assigned to a lower score group if the patient's QS is ≤ a threshold of 18, 19, 20, 21, or 22; and (ii) the patient is assigned to a high score group if the patient's QS is ≥ a threshold of 38, 39, 40, 41, or 42; and (iii) the patient is assigned to an intermediate score group if the patient's QS is >a threshold of 18, 19, 20, 21, or 22 and if the patient does not fall within the high score group.

2. The method of claim 1 further comprising: (e) predicting a likelihood of adverse clinical outcome for the patient based upon the patient's score group, wherein a lower score group indicates a lower risk of adverse clinical outcome than a high score group.

3. The method of claim 1 or 2, wherein, in part (d), the patient is assigned to a high score group if the patient's QS is ≥ 40, preferably wherein the patient is assigned to a high score group if the patient's QS is > 40.

4. The method of claim 1 or 2, wherein, for a patient in the lower score group of part (d)(i), the patient is assigned to a low score group if the patient's QS is ≤ 20 and is assigned to an intermediate score group if the patient's QS is > 20 and if the patient does not fall within the high score group.

5. The method of any one of claims 1-4, wherein the patient is a very low or low, intermediate, or high risk patient, preferably wherein the patient is a very low or low, intermediate, or high risk patient according to one or both of the AUA or NCCN classifications.

6. The method of any one of claims 1-5, wherein the method further comprises providing a report providing the patient's quantitative score and score group.

7. The method of any one of claims 1-6 wherein the levels of the RNA transcripts are normalized against at least one reference gene chosen from ARF1, ATP5E, CLTC, GPS1, and PGK1,

8. The method of any one of claims 1-7 wherein the biological sample is a fresh, frozen, or a fixed, paraffin-embedded sample,

9. The method of any one of claims 1-8 wherein the levels of the RNA transcripts are determined using quantitative reverse-transcriptase polymerase chain reaction (RT-PCR),

10. The method of any one of claims 1-9 wherein the method further comprises determining treatment for the patient based on the patient's quantitative score group,

11. The method of any one of claims 1-10 wherein the adverse clinical outcome is one or more of clinical recurrence (CR), biochemical recurrence (BCR), distant metastasis (Mets), or prostate cancer death (PCD).

12. The method of any one of claims 1-11 wherein the patient is an intermediate risk patient and wherein, if the patient is in the high score group, reclassifying the patient as a high risk patient, and optionally, if the patient is in the lower score group, maintaining the patient's classification as an intermediate risk patient.

13. The method of any one of claims 1-12, further comprising, determining the treatment for the patient based on the patient's quantitative score group, wherein if the patient is in the high score group, determining the patient be treated with multi-modal therapy or with a standard therapy for a high risk patient,
particularly wherein the multi-modal therapy comprises (a) administration of at least one hormonal therapy agent (b) administration of at least one immunotherapy agent, and/or (c) administration of at least one chemotherapy agent.

14. The method of any one of claims 1-12, wherein, if the patient is in the low score group, managing the patient with active surveillance.

15. The method of any one of claims 1-12, further comprising, determining the treatment of an intermediate risk prostate cancer patient, wherein if the patient is in the high score group, determining the patient be treated with multi-modal therapy,
particularly wherein the multi-modal therapy comprises (a) administration of at least one hormonal therapy agent (b) administration of at least one immunotherapy agent, and/or (c) administration of at least one chemotherapy agent.

**Patentansprüche**

1. Verfahren zum Zuordnen eines relativen Risikos eines ungünstigen klinischen Ergebnisses zu einem Prostatakrebspatienten, umfassend:

(a) Messen, in einer biologischen Probe, die aus dem Patienten erhaltene Krebszellen enthält, von Spiegeln von RNA-Transkripten der folgenden Gene: BGN, COL1A1, SFRP4, FLNC, GSN, TPM2, GSTM2, FAM13C, KLK2, AZGP1, SRD5A2 und TPX2;
(b) Normalisieren der Spiegel der RNA-Transkripte der Gene, um normalisierte Genexpressionswerte zu erhalten;
(c) Berechnen eines quantitativen Scores (QS) für den Patienten, wobei der quantitative Score wie folgt berechnet wird, wobei die nachstehenden Gensymbole die normalisierten Genexpressionsniveaus für jedes jeweilige Gen repräsentieren:

(i) Berechnen eines unskalierten quantitativen Scores (QSu) wie folgt:

QSu = 0,735*Stromareaktion-Gruppenscore -0,368* Zellorganisation-Gruppenscore - 0,352*Androgen-Gruppenscore + 0,095*Proliferation-Gruppenscore

Wobei:

Der Stromareaktion-Gruppenscore = 0,527*BGN + 0,457*COL1A1 + 0,156*SFRP4
Der Zellorganisation-Gruppenscore = 0,163*FLNC + 0,504*GSN + 0,421*TPM2 + 0,394*GSTM2
Der Androgen-Gruppenscore = 0,634*FAM13C + 1,079*KLK2 + 0,642*AZGP1 + 0,997*SRD5A2 Thresh
Der Proliferation-Gruppenscore = TPX2 Thresh
wobei der SRD5A2 Thresh und TPX2 Thresh durch Thresholding wie folgt berechnet werden:

$$\text{SRD5A2 Thresh} = \begin{cases} 5.5 & \text{wenn } SRD5A2 < 5.5 \\ SRD5A2 & \text{ansonsten} \end{cases}$$

$$\text{TPX2 Thresh} = \begin{cases} 5.0 & \text{wenn } TPX2 < 5.0 \\ TPX2 & \text{ansonsten} \end{cases}$$

(ii) Berechnen eines skalierten quantitativen Scores (QS), wobei:

$$QS(\text{skaliert}) \left\{ \begin{array}{ll} 0 & \text{wenn } 13.4 \times (QSu+10.5) < 0 \\ 13.4 \times (QSu+10.5) & \text{wenn } 0 \leq 13.4 \times (QSu+10.5) \leq 100 \\ 100 & \text{wenn } 13.4 \times (QSu+10.5) > 100 \end{array} \right.$$

(d) Zuordnen des Patienten zu einer quantitativen Scoregruppe, wobei (i) der Patient zu einer niedrigeren Scoregruppe zugeordnet wird, wenn der QS des Patienten ≤ einem Threshold von 18, 19, 20, 21 oder 22 ist; und (ii) der Patient zu einer hohen Scoregruppe zugeordnet wird, wenn der QS des Patienten ≥ einem Threshold von 38, 39, 40, 41 oder 42 ist; und (iii) der Patient zu einer intermediären Scoregruppe zugeordnet wird, wenn der QS des Patienten > einem Threshold von 18, 19, 20, 21 oder 22 ist und wenn der Patient nicht in die hohe Scoregruppe fällt.

2. Verfahren nach Anspruch, 1 ferner umfassend: (e) Vorhersagen einer Wahrscheinlichkeit eines ungünstigen klinischen Ergebnisses für den Patienten auf Basis der Scoregruppe des Patienten, wobei eine niedrigere Scoregruppe ein niedrigeres Risiko eines ungünstigen klinischen Ergebnisses angibt als eine hohe Scoregruppe.

3. Verfahren nach Anspruch 1 oder 2, wobei, in Teil (d), der Patient zu einer hohen Scoregruppe zugeordnet wird, wenn der QS des Patienten ≥ 40 ist, bevorzugt, wobei der Patient zu einer hohen Scoregruppe zugeordnet wird, wenn der QS des Patienten > 40 ist.

4. Verfahren nach Anspruch 1 oder 2, wobei, bei einem Patienten in der niedrigeren Scoregruppe von Teil (d)(i), der Patient zu einer niedrigen Scoregruppe zugeordnet wird, wenn der QS des Patienten ≤ 20 ist, und einer intermediären Scoregruppe zugeordnet wird, wenn der QS des Patienten > 20 ist und wenn der Patient nicht in die hohe Scoregruppe fällt.

5. Verfahren nach einem der Ansprüche 1-4, wobei der Patient ein Patient mit sehr niedrigem oder niedrigem, intermediärem oder hohem Risiko ist, bevorzugt, wobei der Patient ein Patient mit sehr niedrigem oder niedrigem, intermediärem oder hohem Risiko nach einer oder beiden der AUA- oder NCCN-Klassifikationen ist.

6. Verfahren nach einem der Ansprüche 1-5, wobei das Verfahren ferner das Bereitstellen eines Berichts umfasst, der den quantitativen Score und die Scoregruppe des Patienten bereitstellt.

7. Verfahren nach einem der Ansprüche 1-6, wobei die Spiegel der RNA-Transkripte gegen mindestens ein aus ARF1, ATP5E, CLTC, GPS1 und PGK1 ausgewähltes Referenzgen normalisiert werden.

8. Verfahren nach einem der Ansprüche 1-7, wobei die biologische Probe eine frische, gefrorene oder eine fixierte, in Paraffin eingebettete Probe ist.

9. Verfahren nach einem der Ansprüche 1-8, wobei die Spiegel der RNA-Transkripte unter Verwendung einer quantitativen reversen Transkriptase-Polymerase-Kettenreaktion (RT-PCR) bestimmt werden.

10. Verfahren nach einem der Ansprüche 1-9, wobei das Verfahren ferner das Bestimmen der Behandlung für den Patienten auf Basis der quantitativen Scoregruppe des Patienten umfasst.

11. Verfahren nach einem der Ansprüche 1-10, wobei das ungünstige klinische Ergebnis eines oder mehrere von klinischem Rezidiv (CR), biochemischem Rezidiv (BCR), Fernmetastase (Mets) oder Prostatakrebstod (PCD) ist.

12. Verfahren nach einem der Ansprüche 1-11, wobei der Patient ein Patient mit intermediärem Risiko ist und wobei, wenn der Patient in der hohen Scoregruppe ist, der Patient als ein Patient mit hohem Risiko neu klassifiziert wird und optional, wenn der Patient in der niedrigeren Scoregruppe ist, die Klassifikation des Patienten als ein Patient mit intermediärem Risiko beibehalten wird.

13. Verfahren nach einem der Ansprüche 1-12, ferner umfassend das Bestimmen der Behandlung für den Patienten auf Basis der quantitativen Scoregruppe des Patienten, wobei, wenn der Patient in der hohen Scoregruppe ist, das Bestimmen, dass der Patient mit multimodaler Therapie oder mit einer Standardtherapie für einen Patienten mit hohem Risiko behandelt wird, insbesondere, wobei die multimodale Therapie (a) Verabreichung mindestens eines Hormontherapeutikums (b) Verabreichung mindestens eines Immuntherapeutikums und/oder (c) Verabreichung mindestens eines Chemotherapeutikums umfasst.

14. Verfahren nach einem der Ansprüche 1-12, wobei, wenn der Patient in der niedrigen Scoregruppe ist, Versorgen des Patienten mit aktiver Überwachung.

15. Verfahren nach einem der Ansprüche 1-12, ferner umfassend das Bestimmen der Behandlung eines Patienten mit intermediärem Prostatakrebsrisiko, wobei, wenn der Patient in der hohen Scoregruppe ist, das Bestimmen, dass der Patient mit multimodaler Therapie behandelt wird,
insbesondere, wobei die multimodale Therapie (a) Verabreichung mindestens eines Hormontherapeutikums (b) Verabreichung mindestens eines Immuntherapeutikums und/oder (c) Verabreichung mindestens eines Chemotherapeutikums umfasst.

**Revendications**

1. Procédé d'attribution d'un risque relatif de résultat clinique défavorable à un patient atteint de cancer de la prostate, consistant à :

(a) mesurer, dans un échantillon biologique contenant des cellules cancéreuses obtenues du patient, des niveaux de transcrits d'ARN des gènes suivants : BGN, COL1A1, SFRP4, FLNC, GSN, TPM2, GSTM2, FAM13C, KLK2, AZGP1, SRD5A2 et TPX2 ;
(b) normaliser les niveaux des transcrits d'ARN des gènes afin d'obtenir des niveaux d'expression génique normalisés ;
(c) calculer un score quantitatif (QS) pour le patient, le score quantitatif étant calculé comme suit, où les symboles géniques ci-dessous représentent les niveaux d'expression génique normalisés pour chaque gène respectif :

(i) calculer un score quantitatif non ajusté (QSu) comme suit :

$$QSu = 0{,}735 * \text{score du groupe de réponse stromale} - 0{,}368 * \text{score}$$
$$\text{du groupe d'organisation cellulaire} - 0{,}352 * \text{score du groupe androgène} +$$
$$0{,}095 * \text{score du groupe de prolifération}$$

où :

le score du groupe de réponse stromale = 0,527 * BGN + 0,457 * COL1A1 + 0,156 * SFRP4
le score du groupe d'organisation cellulaire = 0,163 * FLNC + 0,504 * GSN + 0,421 * TPM2 + 0,394 * GSTM2
le score du groupe androgène = 0,634 * FAM13C + 1,079 * KLK2 + 0,642 * AZGP1 + 0,997 * SRD5A2 Thresh
le score du groupe de prolifération = TPX2 Thresh
où SRD5A2 Thresh et TPX2 Thresh sont calculés par seuillage comme suit :

$$\text{SRD5A2 Thresh} = \begin{cases} 5{,}5 & \text{si SRD5A2} < 5.5 \\ \text{SRD5A2} & \text{sinon} \end{cases}$$

$$\text{TPX2 Thresh} = \begin{cases} 5{,}0 & \text{si TPX2} < 5.0 \\ \text{TPX2} & \text{sinon} \end{cases}$$

(ii) calculer un score quantitatif (QS) ajusté où :

$$QS(ajusté) = \begin{cases} 0 & \text{si } 13,4 \times (QSu + 10,5) < 0 \\ 13,4 \times (QSu + 10,5) & \text{si } 0 \leq 13,4 \times (QSu + 10,5) \leq 100 \\ 100 & \text{si } 13,4 \times (QSu + 10,5) > 100 \end{cases}$$

(d) affecter le patient à un groupe de score quantitatif, (i) le patient étant affecté à un groupe de score bas si le QS du patient est ≤ un seuil de 18, 19, 20, 21 ou 22 ; et (ii) le patient étant affecté à un groupe de score haut si le QS du patient est ≥ un seuil de 38, 39, 40, 41 ou 42 ; et (iii) le patient étant affecté à un groupe de score intermédiaire si le QS du patient est > un seuil de 18, 19, 20, 21 ou 22 et si le patient ne tombe pas dans le groupe de score haut.

2. Procédé selon la revendication 1, consistant en outre à : (e) prédire une probabilité de résultat clinique défavorable pour le patient sur la base du groupe de score du patient, un groupe de score bas indiquant un risque de résultat clinique défavorable plus faible qu'un groupe de score haut.

3. Procédé selon la revendication 1 ou 2, dans lequel, à l'étape (d), le patient est affecté à un groupe de score haut si le QS du patient est ≥ 40,
de préférence dans lequel le patient est affecté à un groupe de score haut si le QS du patient est ≥ 40.

4. Procédé selon la revendication 1 ou 2, dans lequel, pour un patient dans le groupe de score bas à l'étape (d)(i), le patient est affecté à un groupe de score bas si le QS du patient est ≤ 20 et est affecté à un groupe de score intermédiaire si le QS du patient est ≥ 20 et si le patient ne tombe pas dans le groupe de score haut.

5. Procédé selon l'une quelconque des revendications 1 à 4, dans lequel le patient est un patient à très bas risque ou à bas risque, à risque intermédiaire ou à haut risque,
de préférence dans lequel le patient est un patient à très bas risque ou à bas risque, à risque intermédiaire ou à haut risque selon la classification AUA et/ou la classification NCCN.

6. Procédé selon l'une quelconque des revendications 1 à 5, le procédé consistant en outre à fournir un rapport fournissant le score quantitatif et le groupe de score du patient.

7. Procédé selon l'une quelconque des revendications 1 à 6, dans lequel les niveaux des transcrits d'ARN sont normalisés par rapport à au moins un gène de référence choisi parmi ARF1, ATP5E, CLTC, GPS1 et PGK1.

8. Procédé selon l'une quelconque des revendications 1 à 7, dans lequel l'échantillon biologique est un échantillon frais, congelé ou fixé et enrobé à la paraffine.

9. Procédé selon l'une quelconque des revendications 1 à 8, dans lequel les niveaux des transcrits d'ARN sont déterminés au moyen d'une réaction en chaîne par polymérase à transcriptase inverse (RT-PCR) quantitative.

10. Procédé selon l'une quelconque des revendications 1 à 9, le procédé consistant en outre à déterminer un traitement pour le patient sur la base du groupe de score quantitatif du patient.

11. Procédé selon l'une quelconque des revendications 1 à 10, dans lequel le résultat clinique défavorable est un ou plusieurs résultats parmi une récurrence clinique (CR), une récurrence biochimique (BCR), des métastases distantes (Mets) et un décès par cancer de la prostate (PCD).

12. Procédé selon l'une quelconque des revendications 1 à 11, le patient étant un patient à risque intermédiaire et le procédé consistant en outre, si le patient est dans le groupe de score haut, à reclasser le patient comme patient à haut risque et éventuellement, si le patient est dans le groupe de score bas, à maintenir la classification du patient comme patient à risque intermédiaire.

13. Procédé selon l'une quelconque des revendications 1 à 12, consistant en outre à déterminer le traitement du patient sur la base du groupe de score quantitatif du patient, et consistant en outre, si le patient est dans le groupe de score haut, à déterminer que le patient doit être traité par thérapie multimodale ou par thérapie standard pour un patient à haut risque,
en particulier la thérapie multimodale comprenant (a) l'administration d'au moins un agent thérapeutique hormonal, (b) l'administration d'au moins un agent immunothérapeutique et/ou (c) l'administration d'au moins un agent chimiothérapeutique.

**14.** Procédé selon l'une quelconque des revendications 1 à 12, le procédé consistant en outre, si le patient est dans le groupe de score bas à gérer le patient avec une surveillance active.

**15.** Procédé selon l'une quelconque des revendications 1 à 12, consistant en outre à déterminer le traitement d'un patient atteint de cancer de la prostate à risque intermédiaire, et consistant en outre, si le patient est dans le groupe de score haut, à déterminer que le patient doit être traité par thérapie multimodale,
en particulier la thérapie multimodale comprenant (a) l'administration d'au moins un agent thérapeutique hormonal, (b) l'administration d'au moins un agent immunothérapeutique et/ou (c) l'administration d'au moins un agent chimiothérapeutique.

Fig. 1A

**PCD**

| NCCN Risk Group | % of Patients | 10-Yr Estimate (95% CI) |
|---|---|---|
| 1 | 3% | 100% (. to .) |
| 2 | 21% | 100% (. to .) |
| 3 | 67% | 99% (98% to 99%) |
| 4 | 9% | 76% (93% to 95%) |

*Fig. 1B*

EP 3 580 357 B1

*Fig. 2A*

Fig. 2B

*Fig. 3*

*Fig. 4*

*Fig. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013116144 A
- US 2013196321 A1
- US 7587279 B
- WO 2006136990 A
- US 9568424 B

### Non-patent literature cited in the description

- **B. HALPERT et al.** *Cancer,* 1963, vol. 16, 737-742
- **B. HOLUND.** *Scand J Urol Nephrol,* 1980, vol. 14, 29-35
- **S. LUNDBERG et al.** *Scand J Urol Nephrol,* 1970, vol. 4, 93-97
- **M. YIN et al.** *J Urol,* 2008, vol. 179, 892-895
- Cancer Facts and Figures. American Cancer Society, 2010
- **JE DAMBER et al.** *Lancet,* 2008, vol. 371, 1710-1721
- **MR COOPERBERG et al.** *J Clin Oncol,* 2010, vol. 28, 1117-1123
- **MR COOPERBERG et al.** *J Clin Oncol,* 2005, vol. 23, 8146-8151
- **AV D'AMICO et al.** *Jama,* 1998, vol. 280, 969-974
- **M HAN et al.** *Urol Clin North Am,* 2001, vol. 28, 555-565
- **WU SHIPLEY et al.** *Jama,* 1999, vol. 281, 1598-1604
- **AJ STEPHENSON et al.** *J Clin Oncol,* 2009, vol. 27, 4300-4305
- **A BILL-AXELSON et al.** *J Natl Cancer Inst,* 2008, vol. 100, 1144-1154
- **J HUGOSSON et al.** *Lancet Oncol,* 2010, vol. 11, 725-732
- **LH KLOTZ et al.** *Can J Urol,* 2006, vol. 13 (1), 48-55
- **S LOEB et al.** *J Clin Oncol,* 2011, vol. 29, 464-467
- **FH SCHRODER et al.** *N Engl J Med,* 2009, vol. 360, 1320-1328
- **LITWIN et al.** *Cancer,* 2007, vol. 109, 2239-2247
- **MG SANDA et al.** *N Engl J Med,* 2008, vol. 358, 1250-1261
- **FR SCHROECK et al.** *Eur Urol,* 2008, vol. 54, 785-793
- **HB CARTER et al.** *J Urol,* 2007, vol. 178, 2359-2364, 2364-2355
- **MA DALL'ERA et al.** *Cancer,* 2008, vol. 112, 2664-2670
- **L KLOTZ et al.** *J Clin Oncol,* 2010, vol. 28, 126-131
- **SL et al.** *J Urol,* 2009, vol. 181, 1628-1633, 1633-1624
- **CR GRIFFIN et al.** *J Urol,* 2007, vol. 178, 860-863
- **PW MUFARRIJ et al.** *J Urol,* 2009, vol. 181, 607-608
- **C. COMPTON et al.** *Arch Pathol Lab Med,* 2000, vol. 124, 979-992
- **E. KLEIN et al.** *Eur Urol,* 2014, vol. 66, 550-560
- **J. CULLEN et al.** *Eur Urol,* 2015, vol. 68, 123-131
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- American Joint Committee on Cancer (AJCC), AJCC Cancer Staging Manual. 2010
- **D. COX.** *Journal of the Royal Statistical Society, Series B,* 1972, vol. 34, 187-220
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **RUPP ; LOCKER.** *Lab Invest.,* 1987, vol. 56, A67
- **DE ANDRÉS et al.** *BioTechniques,* 1995, vol. 18, 42044
- **HELD et al.** *Genome Research,* 1996, vol. 6, 986-994

- **GODFREY et al.** *J. Molec. Diagnostics,* 2000, vol. 2, 84-91
- **SPECHT et al.** *Am. J. Pathol.,* 2001, vol. 158, 419-29
- **KENT, W.J.** *Genome Res.,* 2002, vol. 12 (4), 656-64
- **S. RRAWETZ ; S. MISENER.** Bioinformatics Methods and Protocols: Methods in Molecular Biology. Humana Press, 365-386
- General Concepts for PCR Primer Design. **DIEFFENBACH, CW. et al.** PCR Primer, A Laboratory Manual. Cold Spring Harbor Laboratory Press, 1995, 133-155
- Optimization of PCRs. **INNIS ; GELFAND.** PCR Protocols, A Guide to Methods and Applications. CRC Press, 1994, 5-11
- *Methods Mol. Biol.,* 1997, vol. 70, 520-527
- **DING ; CANTOR.** *Proc. Natl. Acad. Sci. USA,* 2003, vol. 100, 3059-3064
- **OLIPHANT et al.** *Discovery of Markers for Disease (Supplement to Biotechniques),* June 2002
- **FERGUSON et al.** *Analytical Chemistry,* 2000, vol. 72, 5618
- **YANG et al.** *Genome Res.,* 2001, vol. 11, 1888-1898
- **FUKUMURA et al.** *Nucl. Acids. Res.,* 2003, vol. 31 (16), e94
- **SCHENA.** *Proc. Natl. Acad. Sci. USA,* 1996, vol. 93 (2), 106-149
- **VELCULESCU et al.** *Science,* 1995, vol. 270, 484-487
- **VELCULESCU et al.** *Cell,* 1997, vol. 88, 243-51
- **S. BRENNER et al.** *Nature Biotechnology,* 2000, vol. 18 (6), 630-634
- **J. MARIONI.** *Genome Research,* 2008, vol. 18 (9), 1509-1517
- **R. MORIN.** *Genome Research,* 2008, vol. 18 (4), 610-621
- **A. MORTAZAVI.** *Nature Methods,* 2008, vol. 5 (7), 621-628
- **N. CLOONAN.** *Nature Methods,* 2008, vol. 5 (7), 613-619
- **K. ENDERS et al.** *Clin Chem,* 2002, vol. 48, 1647-53
- **R. BOOM et al.** *J Clin Microbiol.,* 1990, vol. 28, 495-503
- **T.E. GODFREY et al.** *J. Molec. Diagnostics,* 2000, vol. 2, 84-91
- **K. SPECHT et al.** *Am. J. Pathol.,* 2001, vol. 158, 419-29
- **M. CRONIN et al.** *Am J Pathol,* 2004, vol. 164, 35-42
- **E. EISENBERG et al.** *Trends in Genetics,* 2003, vol. 19 (7), 362-365